# EUROPÄISCHE PATENTSCHRIFT

(11) **EP 2 678 323 B1**
(45) Veröffentlichungstag und Bekanntmachung des Hinweises auf die Patenterteilung: **10.08.2016**
(21) Anmeldenummer: 12701511.3
(22) Anmeldetag: 27.01.2012
(51) Int. Cl.: C07D 307/68, C08K 5/12

(54) **HEPTYLESTER DER FURANDICARBONSÄURE ALS WEICHMACHER**
FURANDICARBONIC ACID HEPTYL ESTER AS PLASTICIZERS
ESTERS D'ACIDE FURANDICARBOXYLIQUE COMME PLASTIFIANTS

(30) Priorität: 24.02.2011 DE 102011004677
(43) Veröffentlichungstag der Anmeldung: 01.01.2014
(73) Patentinhaber: Evonik Degussa GmbH, 45128 Essen (DE)
(72) Erfinder: BECKER, Hinnerk Gordon, 45257 Essen (DE); GRASS, Michael, 45721 Haltern am See (DE); HUBER, Andre, 45772 Marl (DE); WOELK-FAEHRMANN, Michael, 45768 Marl (DE)
(86) Internationale Anmeldenummer: PCT/EP2012/051325
(87) Internationale Veröffentlichungsnummer: WO 2012/113609

(56) Entgegenhaltungen:
- WO-A1-2011/023590
- JP-A- 2008 127 282
- "Sales Specification EXXAL(TM)7 Isoheptyl Alcohol", , 1. Februar 2006 (2006-02-01), XP55019574, Gefunden im Internet: URL:http://www.imperialoil.ca/Canada-Engli sh/Files/Products_Chemicals/exxal7.pdf [gefunden am 2012-02-16] & DATABASE REGISTRY [Online] CHEMICAL ABSTRACTS SERVICE, COLUMBUS, OHIO, US; 16. November 1984 (1984-11-16), XP002669746, Database accession no. 70914-20-4
- SANDERSON R D ET AL: "SYNTHESIS AND EVALUATION OF DIALKYL FURAN-2,5-DICARBOXYLATES AS PLASTICIZERS FOR PVC", JOURNAL OF APPLIED POLYMER SCIENCE, JOHN WILEY & SONS, INC, US, Bd. 53, Nr. 13, 26. September 1994 (1994-09-26), Seiten 1785-1793, XP000464476, ISSN: 0021-8995, DOI: 10.1002/APP.1994.070531308 in der Anmeldung erwähnt
- YODER P A ET AL: "Ueber Dehydroschleimsäure: eine neue Darstellungsmethode, sowie verschiedene Salze und Ester derselben", BERICHTE DER DEUTSCHEN CHEMISCHEN GESELLSCHAFT ABTEILUNG B:ABHANDLUNGEN, DE, Bd. 34, Nr. 3, 1. Oktober 1901 (1901-10-01), Seiten 3446-3462, XP008148998, ISSN: 0365-9488, DOI: 10.1002/CBER.19010340329 [gefunden am 2006-01-24]

## Beschreibung

Die vorliegende Erfindung betrifft Heptylester der Furandicarbonsäure.

Ein weiterer Gegenstand der Erfindung ist die Verwendung der Heptylester als oder in Weichmachern sowie in Zusammensetzungen, insbesondere in solchen, die Polymere insbesondere PVC enthalten, und ein Herstellungsverfahren für diese Heptylester. Weiterhin sind Gegenstand der Erfindung Kunststoffzusammensetzungen enthaltend diese Heptylester sowie Formkörper oder Folien hergestellt aus, bzw. unter Verwendung dieser Kunststoffzusammensetzungen.

Polyvinylchlorid (PVC) ist eines der wirtschaftlich bedeutendsten Polymere und wird sowohl als Hart-PVC wie auch als Weich-PVC in vielfältigen Anwendungen eingesetzt. Wichtige Anwendungsbereiche sind beispielsweise Kabelummantelungen, Fußbodenbeläge, Tapeten und Rahmen für Kunststofffenster. Zur Erhöhung der Elastizität und zur besseren Verarbeitbarkeit werden dem PVC Weichmacher zugesetzt. Zu diesen üblichen Weichmachern gehören beispielsweise Phthalsäureester wie Di-2-Ethylhexylphthalat (DEHP), Disononylphthalat (DINP) und Diisodecylphthalat (DIDP). Wegen ihrer toxikologischen Eigenschaften ist man bemüht Phthalsäureester durch andere Weichmacher zu ersetzen. Als alternative Weichmacher sind daher in neuerer Zeit beispielsweise Cylclohexandicarbonsäureester beschrieben worden wie Di-isononylcyclohexancarbonsäureester (DINCH).

Weiterhin wurden im Stand der Technik auch Ester der Terephthalsäure als alternative Weichmacher beschrieben.

Bezüglich der Rohstoffbasis liegt die Besonderheit der vorliegenden Erfindung in der optionalen Nutzung nachwachsender Rohstoffe zur Herstellung der erfindungsgemäßen Furandicarbonsäureester. Dabei versteht man im Sinne der vorliegenden Erfindung unter nachwachsenden Rohstoffen im Unterschied zu petrochemischen Rohstoffen, welche auf fossilen Ressourcen, wie z.B. Erdöl oder Steinkohle basieren, solche Rohstoffe, die auf Basis von Biomasse entstehen bzw. hergestellt werden. Die Begriffe "Biomasse", "biobasiert" oder "basierend auf bzw. hergestellt aus nachwachsenden Rohstoffen" umfassen alle Materialien biologischen Ursprungs, die dem sogenannten "Kohlenstoff-Kurzzeitzyklus" entstammen, somit nicht Bestandteil geologischer Formationen oder Fossilschichten sind. Die Identifizierung und Quantifizierung nachwachsender Rohstoffe erfolgt gemäß ASTM-Methode D6866. Kennzeichnend ist u.a. für nachwachsende Rohstoffe ihr Anteil an dem Kohlenstoffisotop ¹⁴C im Gegensatz zu petrochemischen Rohstoffen.

Es ist bekannt, dass mit zunehmender Alkyl-Kettenlänge der Ester deren Unverträglichkeit mit Polymeren, insbesondere mit PVC ansteigt. Dies kann beispielsweise zur Konsequenz haben, dass die PVC-Zusammensetzungen welche derartige Moleküle z.B. als Weichmacher enthalten, atypische und nicht vorhersehbare Viskositätsverläufe zeigen, die die Verarbeitung der PVC-Plastisole erschweren. Bei der Herstellung von Folien zeigt sich oftmals, dass diese zunehmend intransparent erscheinen und/oder eine Verfärbung der Folie eintritt, die sich beispielsweise in einem erhöhten Gelbwert widerspiegelt, der in den meisten Anwendungen unerwünscht ist. Durch eine geringere Verträglichkeit von Weichmachern und PVC wird auch die Permanenz des Weichmachers verringert, d. h., dass diese Weichmacher relativ schnell aus dem PVC-Halbzeug, Fertigzeug bzw. Produkt austreten, was zu einer Versprödung des Produktes führt und damit zu einer starken Funktions- und Wertminderung des entsprechenden Produktes. Man bezeichnet das Verhalten des Weichmachers auch als "Ausbluten" oder "Ausschwitzen".

Andererseits ist auch bekannt, dass Ester mit kurzen Alkylketten in der Regel einerseits eine höhere Flüchtigkeit aufweisen, andererseits aber auch, insbesondere wenn es sich um Ester mit hohem Geliervermögen handelt, bei der Verarbeitung in PVC-Pasten zu Pasten mit geringer Lagerstabilität führen, deren Scherviskosität häufig sehr stark von der Schergeschwindigkeit abhängt, was wiederum zu einer eingeschränkten Verarbeitungsfähigkeit führt.

Bei der Herstellung von PVC-Plastisolen ist insbesondere darauf zu achten, dass bei der Verarbeitung eine möglichst niedrige Viskosität eingehalten wird, um eine gleichmäßige Verteilung des Weichmachers im PVC zu erreichen. Darüber hinaus ist auch eine hohe Lagerstabilität des PVC-Plastisols, sowie eine geringe Abhängigkeit der Scherviskosität der Paste von der Schergeschwindigkeit erwünscht. Aus PVC-Plastisolen hergestellte (ungefüllte) Folien sollen transparent sein und einen möglichst niedrigen Gelbwert aufweisen. Der Weichmacher soll weiterhin eine hohe Permanenz aufweisen.

Im Stand der Technik sind verschiedene alternative Weichmacher bekannt geworden für den Einsatz in PVC. Die EP 1 808 457 A1 beschreibt den Einsatz von Terephthalsäuredialkylestern, die dadurch gekennzeichnet sind, dass die Alkylreste eine längste Kohlenstoffkette von mindestens 4 Kohlenstoffatomen aufweisen und eine Gesamtzahl an Kohlenstoffatomen pro Alkylrest von 5 aufweisen. Es wird weiterhin beschrieben, dass Terephthalsäureester mit 4 bis 5 Kohlenstoffatomen in der längsten Kohlenstoffkette des Alkohols als schnell gelierende Weichmacher für PVC gut geeignet sind.

In ähnlicher Weise beschreibt die WO 2009/095126 A1 Gemische von Di-isononylestern der Terephthalsäure sowie Verfahren zu ihrer Herstellung. Diese Weichmacher weisen einen durchschnittlichen Verzweigungsgrad der Isononylreste auf, der im Bereich von 1,0 bis 2,2 liegt und werden ebenfalls als Weichmacher für PVC eingesetzt.

Terephthalsäurester von Alkoholen mit 7 Kohlenstoffatomen und ihre Verwendung als Weichmacher werden z.B. in der WO 2010/071717 A1 beschrieben.

Viele Ester der Furandicarbonsäure, wie beispielsweise Di-n-butylfuran-2,5-Dicarboxylate und Di-n-hexylfuran-2,5-Dicarboxylate sind bei Raumtemperatur kristalline Feststoffe, die sich aufgrund ihrer Feststoffeigenschaft nicht bzw. nur schwer für die Herstellung von flüssigen Zusammensetzungen, insbesondere von (Polymer-) Plastisolen einsetzen lassen. So ist die Herstellung von Polymerpasten bzw. Plastisolen im technischen Maßstab nur mit flüssigen Weichmachern zu realisieren. Feste Weichmacher müssen vorher in entsprechenden Lösungsmitteln gelöst werden, was das Verfahren aufwendig und teuer macht.

Die technische Aufgabe der Erfindung war es daher, Verbindungen zur Verfügung zu stellen, die als oder in Weichmachern eingesetzt werden können, welche sich auch in Plastisolen verarbeiten lassen, gute Geliereigenschaften besitzen, in Plastisolen eine geringe Abhängigkeit der Pastenviskosität von der Schergeschwindigkeit zeigen und einen guten Gelbwert und hohe Transparenz bei der Verarbeitung zu Folien aufweisen. Eine zusätzliche Aufgabe war es die Lösung dieser technischen Problemstelllung in Zusammenhang mit einer Substanz bzw. Verbindung zu lösen, welche zumindest teilweise aus nachwachsenden Rohstoffen hergestellt werden kann.

Diese technische Aufgabe wird gelöst durch Heptylester der Furandicarbonsäure.

Vorzugsweise handelt es sich bei den Heptylestern um Diheptylester.

Die technische Aufgabe wird insbesondere durch Heptylester der Furandicarbonsäure gelöst, welche mindestens eine der folgenden Eigenschaften aufweisen:
1) die Dichte bei 20 °C beträgt maximal 1,1 g/cm³
2) die Eigenviskosität bei 25 °C beträgt maximal 120 mPa*s
3) beim Vermessen mit einem Differentialkalorimeter tritt kein Schmelzsignal bei Temperaturen > 20 °C auf.

In einer weiteren Ausführungsform weist der Heptylester mindestens zwei der oben genannten Eigenschaften auf.

Überraschenderweise wurde festgestellt, dass solche erfindungsgemäßen Heptylester im Gegensatz zu den entsprechenden homologen Butyl- und Hexylestern keine Feststoffe sind und als Flüssigkeiten bei Raumtemperatur gut zu verarbeiten sind. Die entsprechenden homologen Di-n-butylfurandicarboxylate und Di-n-hexylfurandicarboxylate sind bei Raumtemperatur fest und besitzen Schmelzpunkte im Bereich von 30-40 °C. Diese können daher nicht im technischen Maßstab zur Herstellung von Polymerpasten bzw. Plastisolen eingesetzt werden.

Di-n-butyl-furandicarboxylat und Di-n-hexyl-furandicarboxylat sind aus den Arbeiten von Sanderson et al (R. D. Sanderson, D.F.Schneider, I. Schreuder; J.Appl.Polym.Sci.; 53 (1991); 1785-1793) bekannt. Es handelt sich dabei um kristalline Feststoffe mit Schmelzpunkten von ca. 42 °C (Di-n-butyl-furandicarboxylat) und ca. 32 °C (Di-n-hexyl-furandicarboxylat), die für zahlreiche Anwendungen wie z.B. die Herstellung von Polymerpasten nicht sinnvoll einsetzbar sind.

Diheptylester der Furandicarbonsäure sind bislang noch nicht als beschrieben worden, insbesondere gibt es keine Hinweise zur Verwendung von Diheptylestern der Furandicarbonsäure in Polymerzusammensetzungen und/oder als Weichmacher.

Überraschenderweise wurde nun gefunden, dass die erfindungsgemäßen Heptylester in flüssiger Form ohne Verfestigung hergestellt werden können, und sich vorteilhaft als Komponente in Polymerformulierungen, z.B. als Weichmacher in PVC-Formulierungen einsetzen lassen.

Es wurde weiterhin festgestellt, dass die erfindungsgemäßen Heptylester exzellente Geliereigenschaften aufweisen, wenn sie mit PVC verarbeitet werden.

Die PVC-Pasten auf Basis der erfindungsgemäßen Heptylester weisen nur eine geringe Abhängigkeit der Pastenviskosität von der Schergeschwindigkeit auf. Sie sind damit in einem weiten Schergeschwindigkeitsbereich und mit unterschiedlichsten Verarbeitungsmethoden verarbeitbar.

Aufgrund des günstigen Gelierverhaltens können die entsprechenden PVC-Pasten schneller bzw. bei niedrigeren Temperaturen verarbeitet werden.

Durch Abmischung mit primären oder sekundären nicht phthalathaltigen Weichmachern wie z.B. Diisononylcyclohexancarbonsäureester (DINCH), welches eine deutlich schlechtere weichmachende Wirkung aufweist, wird ferner die Weichmachereffizienz der erfindungsgemäßen Heptylester der Furandicarbonsäure (DIHFDC) nur unwesentlich verschlechtert.

Es wurde auch festgestellt, dass die Prüfkörper, die einen erfindungsgemäßen Heptylester enthalten, bei erhöhter Temperatur deutlich geringere Masseverluste aufweisen als Prüfkörper, die als Weichmacher Hexylester der Furandicarbonsäure enthalten. Die Prüfkörper mit den erfindungsgemäßen Heptylestern weisen somit eine deutlich bessere Beständigkeit bei erhöhter Temperatur auf.

Ein weiterer Vorteil des erfindungsgemäßen Weichmachers liegt darin, dass Proben, die den erfindungsgemäßen Weichmacher enthalten, eine deutlich bessere Thermostabilität aufweisen als Proben welche Diisononylcyclohexancarbonsäureester (DINCH) als alleinigen Weichmacher, Di-n-hexyl-furandicarboxylat (DNHFDC) als alleinigen Weichmacher sowie Di-(2-ethylhexyl)- furandicarboxylat (D2EHFDC) in Abmischung mit DINCH enthalten.

Prüfkörper welche die erfindungsgemäßen Heptylester enthalten, zeigen zu dem im Bereich > 0 °C eine deutlich höhere Flexibilität sowohl als Prüfkörper welche das lineare DNHFDC enthalten als auch als Prüfkörper, welche das einfach verzweigte und um ein C-Atom "längere" D2EHFDC enthalten. Die erfindungsgemäße Probe weist außerdem auch bei 0 °C und bei -30 °C eine ebenfalls deutlich höhere Flexibilität im Vergleich mit der D2EHFDC-Probe auf. Dies ist umso erstaunlicher, als die Glasübergangstemperatur der erfindungsgemäßen Probe im Vergleich mit den beiden anderen Proben höher liegt, und die Flexibilität von Weich-PVC-Prüfkörpern in der Regel mit abnehmender Glasübergangstemperatur steigt.

Es wurde weiterhin festgestellt, dass auch mit transparenten Folien aus PVC, die den erfindungsgemäßen Weichmacher enthalten, Produkte erhalten werden, die eine sehr hohe Transparenz aufweisen, die zum Teil höher ist, als von Folien, die mit Dibutylterephthalat als Weichmacher hergestellt wurden.

Ein besonderer ökonomischer und gleichzeitig ökologischer Vorteil der vorliegenden Erfindung liegt in der gleichzeitigen Nutzung von nachwachsenden und petrochemischen Rohstoffen für die Herstellung der erfindungsgemäßen Furandicarbonsäureester, was einerseits eine besonders preiswerte Herstellung und eine breite Anwendbarkeit ermöglicht, andererseits aber auch zu besonders "nachhaltigen" Produkten führt.

In einer bevorzugten Ausführungsform ist der Heptylester der Furandicarbonsäure ein Diheptylfuran-2,5-Dicarboxylat. Dieses kann auch in Form von mindestens zwei isomeren Diheptylfuran-2,5-Dicarboxylaten vorhanden sein.

In einer bevorzugten Ausführungsform werden daher auch Weichmacher eingesetzt aus isomeren Diheptylfuran-2,5-dicarboxylaten, wobei diese isomere Heptylgruppen enthalten, die insbesondere ausgewählt sind aus n-Heptyl-, 2-Methylhexyl-, 3-Methylhexyl-, 4-Methylhexyl-, 5-Methylhexyl-, 2,2-Dimethylpentyl-, 2,3-Dimethylpentyl-, 3,3-Dimethylpentyl-, 2,4-Dimethylpentyl-, 3-Ethylpentyl-, 2,2,3-Trimethylbutyl- oder 2-Ethyl-3-methylbutyl-Gruppen.

In einer besonderen Ausführungsform bestehen (unabhängig von ihrer Konformation) mindestens 70 mol% aller Alkylesterketten aus 7 Kohlenstoffatomen, bevorzugt mindestens 80 mol%, besonders bevorzugt mindestens 90 mol% und insbesondere bevorzugt mindestens 92 mol%. Bei den Alkylketten, die weniger oder mehr als 7 Kohlenstoffatome aufweisen handelt es sich besonders bevorzugt um solche, mit 6 oder 8 Kohlenstoffatomen. Insbesondere bevorzugt handelt es sich um 2,3-Dimethylbutyl-, 2-Ethylbutyl-, 4-Methylpentyl- oder 3-Methylpentyl-Gruppen.

In einer bevorzugten Ausführungsform besitzt keiner der isomeren Diheptylfuran-2,5-dicarboyxlaten einen Anteil von mehr als 90 Gew.-% im isomeren Estergemisch.

Die Bestimmung der prozentualen Verteilung der C7-Alkyl-Reste kann auf einfache Weise durch Verseifen der Ester, Abtrennen des erhaltenen Alkohols und gaschromatographische (GC) Analyse des Alkohols erfolgen. Beispielsweise kann die gaschromatographische Trennung auf einer Polydimethylsiloxan-Säule (z. B. DB 5) als stationärer Phase mit einer Länge von 60 m, einem inneren Durchmesser von 0,25 mm und einer Filmdicke von 0,25 µm durchgeführt werden.

Es können vorzugsweise weitere zusätzliche Weichmacher, ausgenommen Heptylester der Furandicarbonsäure enthalten sein.

Solche zusätzlichen Weichmacher sind beispielsweise ausgewählt aus folgender Liste:
Phthalsäuredialkylester, bevorzugt mit 4 bis 13 C-Atomen in der Alkylkette; Trimellitsäuretrialkylester, bevorzugt mit 4 bis 9 C-Atomen in der Seitenkette; Adipinsäuredialkylester, bevorzugt mit 4 bis 9 C-Atomen in der Seitenkette; Terephthalsäuredialkylester, jeweils bevorzugt mit 4 bis 13 C-Atomen, insbesondere 4 bis 9 C-Atomen in der Seitenkette; 1,2-Cyclohexandisäurealkylester, 1,3-Cyclohexandisäurealkylester und 1,4-Cyclohexandisäurealkylester, hierbei bevorzugt 1,2-Cyclohexandisäurealkyl-ester, jeweils bevorzugt mit 4 bis 10 Kohlenstoffatomen in der Seitenkette; Dibenzoesäurester von Glykolen; Alkylsulfonsäureester von Phenol mit vorzugsweise einem Alkylrest, der 8 bis 22 C-Atome enthält; Glycerinester; Isosorbidester, insbesondere Isosorbiddialkanoate; epoxidierte Pflanzenöle insbesondere epoxidiertes Sojabohnenöl; gesättigte oder ungesättigte Fettsäureester, welche (im ungesättigten Fall) ganz oder teilweise epoxidiert sein können; Citronensäuretriester mit freier oder carboxylierter OH-Gruppe und beispielsweise Alkylresten von 4 bis 8 C-Atomen, Alkylpyrrolidonderivate mit Alkylresten von 4 bis 18 C-Atomen sowie Benzoesäurealkylester, vorzugsweise mit 7 bis 13 C-Atomen in der Alkylkette. In allen Fällen können die Alkylreste linear oder verzweigt und gleich oder verschieden sein.

Besonders bevorzugt wird in den erfindungsgemäßen Mischungen kein *ortho*-Phthalat als zusätzlicher Weichmacher, eingesetzt.

In einer besonderen Ausführungsform handelt es sich bei mindestens einem der in der erfindungsgemäßen Zusammensetzung verwendeten zusätzlichen Weichmacher um einen Trimellitsäuretrialkylester. Bevorzugt weist dieser Trimellitsäuretrialkylester Esterseitenketten mit 4 bis 9 Kohlenstoffatomen auf, wobei die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 8 Kohlenstoffatomen pro Estergruppe, insbesondere bevorzugt um eine Gruppe mit maximal 7 Kohlenstoffatomen und ganz besonders bevorzugt um eine Gruppe mit maximal 6 Kohlenstoffatomen. Die Kombination der erfindungsgemäßen Furandicarbonsäurediheptylester mit Trimellitsäuretrialkylestern führt bei Verwendung in PVC-Plastisolen insbesondere zu Produkten, die einen niedrigen Anteil an flüchtigen Bestandteilen und gute Temperaturbeständigkeit aufweisen.

In einer weiteren besonderen Ausführungsform handelt es sich bei mindestens einem der in der erfindungsgemäßen Zusammensetzung verwendeten zusätzlichen Weichmacher, um ein Adipinsäuredialkylester. Bevorzugt weist dieser Adipinsäuredialkylester Esterseitenketten mit 4 bis 9 Kohlenstoffatomen auf, wobei auch hier die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 8 Kohlenstoffatomen pro Estergruppe, insbesondere bevorzugt um eine Gruppe mit maximal 7 Kohlenstoffatomen und ganz besonders bevorzugt um eine Gruppe mit maximal 6 Kohlenstoffatomen. Insbesondere handelt es sich bei mindestens einem der verwendeten Adipinsäuredialkylester um Dioctyladipat. Die Kombination der erfindungsgemäßen Furandicarbonsäurediheptylester mit Adipinsäuredialkylestern führt bei Verwendung in PVC-Plastisolen insbesondere zu Produkten, die eine niedrige Plastisolviskosität sowie im verarbeiteten Zustand gute Tieftemperatureigenschaften (z.B. eine sehr niedrige Glasübergangstemperatur) aufweisen.

In einer weiteren besonderen Ausführungsform handelt es sich bei mindestens einem der in der erfindungsgemäßen Zusammensetzung verwendeten zusätzlichen Weichmacher, um ein Terephthalsäuredialkylester. Bevorzugt weist dieser Terephthalsäuredialkylester Esterseitenketten mit 4 bis 13 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 10 Kohlenstoffatomen pro Estergruppe, insbesondere bevorzugt um eine Gruppe mit maximal 9 Kohlenstoffatomen und ganz besonders bevorzugt um eine Gruppe mit maximal 8 Kohlenstoffatomen. Insbesondere handelt es sich bei mindestens einem der verwendeten Terephthalsäuredialkylester um Di-(isononyl)terephthalat, Di-(2-ethylhexyl)terephthalat, Di-*n*-heptylterephthalat, Di-i*so*-heptylterephthalat, Di-*n-*butylterephthalat, Di-(3-methylbutyl)terephthalat oder Di-*n*-pentylterephthalat. Die Kombination der erfindungsgemäßen Furandicarbonsäurediheptylester mit Terephthalsäuredialkylestern führt bei Verwendung in PVC-Plastisolen insbesondere zu Produkten, die (je nach Esterkettenlänge der verwendeten Terephthalsäuredialkylester) eine sehr gute Thermostabilität und gute Tieftemperatureigenschaften bei gleichzeitig geringen flüchtigen Bestandteilen aufweisen.

In einer weiteren besonderen Ausführungsform handelt es sich bei mindestens einem der in der erfindungsgemäßen Zusammensetzung verwendeten zusätzlichen Weichmacher, um einen Dialkylester der Cyclohexandicarbonsäure, besonders bevorzugt um einen Dialkylester der 1,2-Cyclohexandicarbonsäure. Bevorzugt weist dieser Cyclohexandicarbonsäuredialkylester Esterseitenketten mit 4 bis 10 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Cyclohexandicarbonsäuredialkylester um 1,2-Cyclohexansäuredi-isononylester, 1,2-Cyclohexansäuredi-2-ethylhexylester, 1,2-Cyclohexansäuredi-*n*-pentylester, 1,2-Cyclohexan-säuredi-*n*-heptylester, 1,2-Cyclohexansäuredi-*iso*-heptylester, 1,2-Cyclohexansäuredi-*n*-butylester, 1,4-Cyclohexansäuredi-*n*-butylester, 1,3-Cyclohexansäuredi-*n-*butylester oder 1,2-Cyclohexansäuredi(-3-methylbutyl)ester. Die Kombination der erfindungsgemäßen Furandicarbonsäurediheptylester mit Dialkylestern der Cyclohexandicarbonsäure führt bei Verwendung in PVC-Plastisolen insbesondere zu Produkten, die sich insbesondere durch eine sehr niedrige Plastisolviskosität bei gleichzeitig guten Geliereigenschaften auszeichnen.

In einer weiteren besonderen Ausführungsform handelt es sich bei mindestens einem der in der erfindungsgemäßen Zusammensetzung verwendeten zusätzlichen Weichmacher, um einen Glycerinester, besonders bevorzugt um einen Glycerintriester. Die Estergruppen können dabei sowohl von aliphatischer als auch aromatischer Struktur sein. Bevorzugt weist dieser Glycerinester Esterseitenketten mit 1 bis 24 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei einer der Estergruppen um Hydroxystearinsäure, wobei die Hydroxyfunktion bevorzugt ebenfalls verestert ist, besonders bevorzugt durch eine Acetylgruppe Weiterhin besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Glycerinester um ein Glycerintriacetat. Die Kombination der erfindungsgemäßen Furandicarbonsäurediheptylester mit Glycerinestern führt insbesondere zu besonders nachhaltigen Produkten welche zu einem großen Anteil auf Basis nachwachsender Rohstoffe hergestellt werden können.

In einer weiteren besonderen Ausführungsform handelt es sich bei mindestens einem der in der erfindungsgemäßen Zusammensetzung verwendeten zusätzlichen Weichmacher, um einen Citronensäuretriester mit freier oder carboxylierter OH-Gruppe. Die Estergruppen können dabei auch hier sowohl von aliphatischer als auch aromatischer Struktur sein. Insbesondere bevorzugt handelt es sich um einen Citronensäuretrialkylester mit carboxylierter OH-Gruppe. Bevorzugt weist dieser Citronensäuretrialkylester Esterseitenketten mit 1 bis 9 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Citronensäureestern um Acetyl-tributylcitrat, Acetyl-tri-*n*-butylcitrat, Acetyl-tri-*n*-pentylcitrat oder Acetyl-tri-*iso*-heptylcitrat. Die Kombination der erfindungsgemäßen Furandicarbonsäurediheptylester mit Citronensäuretriestern mit freier oder carboxylierter OH-Gruppe führt insbesondere zu Plastisolen, die eine besonders gute Gelierfähigkeit insbesondere bei niedrigen Temperaturen besitzen, und ebenso wie die Kombination mit Citronensäureestern hinsichtlich der zur Herstellung verwendeten nachwachsenden Rohstoffe als besonders nachhaltig einzustufen sind.

In einer bevorzugten Ausführungsform liegt das Massenverhältnis aus eingesetzten zusätzlichen Weichmachern, zum erfindungsgemäßen Heptylestern zwischen 1:20 und 20:1, bevorzugt zwischen 1:15 und 15:1, besonders bevorzugt zwischen 1:10 und 15:1 und insbesondere besonders bevorzugt zwischen 1:10 und 10:1.

Die erfindungsgemäßen Heptylester bzw. die aus ihnen hergestellten Weichmacher können in allen möglichen Darreichungsformen vorliegen, z.B. als Flüssigkeit, insbesondere als (bei Raumtemperatur) pumpbare Flüssigkeit, als Paste, Schutzmasse, Plastisol, Pulver, Feststoff oder Festkörper. Insbesondere bevorzugt liegen sie als Flüssigkeit und insbesondere bevorzugt als (bei Raumtemperatur) pumpbare Flüssigkeit vor.

Neben den Heptylestern selbst wird auch ein Verfahren zu deren Herstellung beansprucht.

Verfahren zur Herstellung eines zuvor beschriebenen Heptylesters umfassend die Verfahrensschritte:
a) In Kontakt bringen von Furandicarbonsäure und/oder mindestens eines Furandicarbonsäurederivates, insbesondere von Furandicarbonsäuredimethylester oder Furandicarbonsäuredichlorid, mit einem oder mehreren aliphatischen Alkoholen mit 7-Kohlenstoffatomen, bzw. einem Alkoholgemisch aliphatischer Alkohole die zu > 80 mol% 7-Kohlenstoffatome aufweisen, sowie optional einem oder mehreren Veresterungskatalysatoren und/oder weiteren Substanzen,
b) Erhitzen der beschriebenen Reaktionsmischung auf eine Temperatur > 50 °C und Veresterung bzw. Umesterung unter Abtrennung mindestens einer niedermolekularen Substanz aus dem Reaktionsgemisch, wobei das Abtrennen in Verfahrensschritt b) vorzugsweise thermisch erfolgt.

In einem alternativen Verfahren zur Herstellung eines zuvor beschriebenen Heptylesters, umfasst dieses die Verfahrensschritte:
a) In Kontakt bringen von 5-Hydroxymethylfurfural und / oder mindestens eines Furanderivates mit einem oder mehreren aliphatischen Alkoholen mit 7-Kohlenstoffatomen, sowie mindestens einem Katalysator und mindestens einer sauerstoffhaltigen Komponente,
b) Temperieren der beschriebenen Reaktionsmischung auf eine Temperatur > 0 °C und Durchführung einer oxidativen Veresterung, wobei unter dem Begriff der "oxidativen Veresterung" die (beliebige) Kombination aus Oxidation und Veresterung in bevorzugt einem Verfahrensschritt, insbesondere bevorzugt in einem Reaktionsraum zu verstehen ist.

Letzteres Verfahren ist hierbei bevorzugt.

Die Heptylester können hergestellt werden durch "direkte" Veresterung der Furandicarbonsäure oder durch Umesterung beispielsweise aus den Methylestern der Furandicarbonsäure.

Zur Herstellung der erfindungsgemäßen Heptylester mittels Veresterung wird entweder Furandicarbonsäure oder ein reaktives Derivat wie beispielsweise das entsprechende Dichlorid mit einem oder mehreren aliphatischen Alkoholen mit 7-Kohlenstoffatomen umgesetzt. Bevorzugt erfolgt die Veresterung ausgehend von Furandicarbonsäure und einem oder mehreren aliphatischen Alkoholen mit 7-Kohlenstoffatomen mit Hilfe eines Katalysators.

Die Veresterung der Furandicarbonsäure mit einem oder mehreren aliphatischen Alkoholen mit 7-Kohlenstoffatomen zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen Säure und Alkohol und den Produkten Ester und Wasser. Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkohole niedriger als die Furandicarbonsäure, deren reaktive Derivate und deren Ester sieden, werden sie häufig als Schleppmittel eingesetzt, das nach Wasserabtrennung wieder in den Prozess zurückgeführt werden kann.

Der zur Bildung des Esters eingesetzte Alkohol bzw. das isomere Heptanolgemisch, das gleichzeitig als Schleppmittel dient, wird im Überschuss von bevorzugt 5 bis 50 Massen-%, insbesondere 10 bis 30 Massen-% der zur Bildung des Esters notwendigen Menge eingesetzt.

Als Veresterungskatalysatoren können Säuren, z.B. Brönstedt-Säuren wie beispielsweise Schwefelsäure, Methansulfonsäure oder p-Toluolsulfonsäure, oder Metalle oder deren Verbindungen (in der Regel Lewissäuren) eingesetzt werden. Geeignet sind z. B. Zinn, Titan, Zirkonium, die als fein verteilte Metalle oder zweckmäßig in Form ihrer Salze (z.B. Halogenide), Oxide oder löslicher oder unlöslicher organischer Verbindungen verwendet werden. Die Metallkatalysatoren sind im Gegensatz zu Protonensäuren Hochtemperaturkatalysatoren, die ihre volle Aktivität oft erst bei Temperaturen oberhalb 180 °C erreichen. Hierbei ist jedoch zu beachten, dass die Furandicarbonsäure bei Temperaturen oberhalb von 190 °C zur Abspaltung von CO₂ (Decarboxylierung) neigt, und sich hieraus dann die Monocarbonsäure bildet, welche nicht mehr zum Zielprodukt umgesetzt werden kann.

Die Metallkatalysatoren werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraheptylorthotitanat, Tetraisopropylorthotitanat oder Tetrabutylorthotitanat sowie Zirkoniumester wie Tetraheptylzirkonat oder Tetrabutylzirkonat.

Die Katalysatorkonzentration hängt von der Art des Katalysators ab. Bei den bevorzugt eingesetzten Titanverbindungen beträgt diese 0,005 bis 2,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren insbesondere zwischen 160 °C und 270 °C, vorzugsweise bei 160 °C bis 200 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei vermindertem Druck durchgeführt. Beispielsweise wird bei der Umsetzung von Furandicarbonsäure mit einem Gemisch isomerer Heptanole in einem Temperaturbereich von 160 °C bis 190 °C im Druckbereich von 0,1 MPa bis 0,001 MPa gearbeitet.

Die in die Reaktion zurückzuführende Flüssigkeitsmenge kann teilweise oder vollständig aus Alkohol bestehen, der durch Aufarbeitung des Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereit stehenden Alkohol zu ersetzen.

Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und ggf. Leichtsieder, Neutralisation der vorhandenen Säuren, optional eine Wasserdampfdestillation, Umwandlung des Katalysators in einen leicht filtrierbaren Rückstand, Abtrennung der Feststoffe und gegebenenfalls eine Trocknung. Dabei können je nach angewendetem Aufarbeitungsverfahren die Reihenfolge dieser Schritte verschieden sein. Optional kann das Reaktionsprodukt aus dem Reaktionsgemisch, gegebenenfalls nach Neutralisation des Ansatzes, destillativ abgetrennt werden.

Alternativ können die erfindungsgemäßen Heptylester durch Umesterung eines Furan-2,5-dicarbonsäurediesters mit einem oder mehreren aliphatischen Alkoholen mit 7-Kohlenstoffatomen gewonnen werden. Als Edukte werden bevorzugt Furan-2,5-dicarbonsäurediester eingesetzt, deren am O-Atom der Estergruppe gebundenen Alkylreste 1-4 C-Atome aufweisen. Diese Reste können aliphatisch, geradkettig oder verzweigt, alicyclisch oder aromatisch sein. Eine oder mehrere Methylengruppen dieser Alkyl-Reste können durch Sauerstoff substituiert sein. Es ist zweckmäßig, dass die dem Eduktester zugrunde liegenden Alkohole niedriger sieden als das/die eingesetzte(n) Heptanol(e). Ein bevorzugter Einsatzstoff ist Furan-2,5-dicarbonsäuredimethylester.

Die Verwendung eines Furan-2,5-dicarbonsäurediesters zur Herstellung der erfindungsgemäßen Furandicarbonsäurediheptylester ist in sofern besonders vorteilhaft, weil die Furan-2,5-dicarbonsäurediester im Gegensatz zur Furan-2.5-dicarbonsäure selbst in der Regel eine höhere thermische Stabilität aufweisen und insbesondere auch unzersetzt durch thermische Trennverfahren (z.B. Destillation) aufgereinigt werden können.

Die Umesterung kann katalytisch, beispielsweise mit Brönstedt- oder Lewissäuren oder Basen, durchgeführt werden. Ganz gleich welcher Katalysator eingesetzt wird, es entsteht immer ein temperaturabhängiges Gleichgewicht zwischen den Einsatzstoffen (Dialkylester und Heptanol bzw. Heptanolgemisch) und den Produkten (Diheptylester bzw. Diheptylestergemisch und freigesetzter Alkohol). Um das Gleichgewicht zu Gunsten des Diheptylesters bzw. des Diheptylestergemisches zu verschieben, wird der aus dem Eduktester entstehende Alkohol aus dem Reaktionsgemisch abdestilliert.

Es ist auch hier zweckmäßig, das Heptanol bzw. das Heptanolgemisch im Überschuss einzusetzen.

Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirkonium, die als fein verteilte Metalle oder zweckmäßig in Form ihrer Salze (z.B. Halogenide), Oxide oder löslichen oder unlösliche organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraheptylorthotitanat, Tetraisopropylorthotitanat oder Tetrabutylorthotitanat sowie Zirkoniumester wie Tetraheptylzirkonat oder Tetrabutylzirkonat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem Heptanol bzw. einem isomeren Heptanolgemisch hergestellt werden.

Die Katalysatorkonzentration hängt von der Art des Katalysators ab. Sie liegt üblicherweise zwischen 0,005 bis 2,0 Massen-% bezogen auf das Reaktionsgemisch.

Die Reaktionstemperaturen für die Umesterung liegen üblicherweise zwischen 50 °C und 220 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden.

Neben der direkten Veresterung und der Umesterung können die erfindungsgemäßen Furandicarbonsäurediheptylester auch über die sogenannte oxidative Veresterung hergestellt werden. Dies hat insbesondere den Vorteil, dass die Zwischenstufe der Furandicarbonsäure bzw. des Furandicarbonsäureesters nicht separiert werden muss, sondern direkt mit der (semistabilen) Zwischenstufe z.B. des 5-Hydroxymethylfurfurals bzw. eines anderen Furanderivates gearbeitet werden kann. Hinzu kommt, dass in der Regel niedrige Temperaturen (d.h. geringere Tendenz zur Bildung von Nebenprodukten) und relativ kurze Reaktionszeiten möglich sind.

Um die Oxidationsreaktion zu ermöglichen muß eine sauerstoffhaltige Komponente im Reaktionsgemisch vorhanden sein. Besonders vorteilhaft eignen sich hierfür Sauerstoff, Luft und/oder Peroxide, insbesondere Wasserstoffperoxid.

Die oxidative Veresterung wird besonders bevorzugt in Anwesenheit eines Katalysators durchgeführt, der die Reaktionszeit signifikant herabsetzt. Dabei kann es sich sowohl um einen homogenen wie auch um einen heterogenen Katalysator handeln. Der Katalysator -bei hetereogenen Katalysatoren die aktive Katalysatoroberfläche- besitzt besonders bevorzugt eine Lewisacidität. Bevorzugt handelt es sich um einen Edelmetallkatalysator, im Fall eines heterogenen Katalysators um einen Edelmetallkatalysator mit nanoskaliger Oberfläche, insbesondere um einen Goldkatalysator mit nanoskaliger Oberfläche. Bei heterogenen Katalysatoren ist insbesondere die Verwendung eines anorganischen Katalysatorträgers von Vorteil, besonders bevorzugt werden makroporöse oder mikroporöse Träger, insbesondere solche, die nanoskalig strukturierte Porenoberflächen aufweisen.

Neben den Heptylestern wird auch deren Verwendung als oder in Weichmacher(n) für Polymere beansprucht. Bei dem Polymer handelt es sich vorzugsweise um ein PVC.

Des weiteren wird die Verwendung der erfindungsgemäßen Heptylester in Klebstoffen, Dichtungsmassen, Beschichtungsmassen, Lacken, Farben, Plastisolen, Pasten, Kunstleder, Fußbodenbeläge, Unterbodenschutz, Gewebebeschichtungen, Tapeten oder Tinten beansprucht.

Des Weiteren werden Kunststoffe bzw. Kunststoffzusammensetzungen beansprucht, welche mindestens einen der zuvor beschriebenen Heptylester bzw. Weichmacher enthalten.

Die erfindungsgemäßen Weichmacher können in Kunststoffzusammensetzungen eingesetzt werden, die mindestens ein Polymer enthalten. Die Polymere sind dabei vorzugsweise ausgewählt aus der Gruppe bestehend aus:
Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat-Copolymere (ASA), Styrolacrylonitril-Copolymere (SAN), Acrylonitril-Butadien-Styrol-Copolymere (ABS), Styrol-Maleinsäureanhydrid-Copolymere (SMA), Styrol-Methacrylsäure-Copolymere, Polyolefine und Polyolefincopolymere, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten aufweisen. Vorzugsweise enthalten die erfindungsgemäßen Polymerzusammensetzungen PVC oder Homo- oder Copolymere auf Basis von Ethylen, Propylen, Butadien, Vinylacetat, Glycidylacrylat, Glycidylmethacrylat, Methacrylaten, Ethylacrylaten, Butylacrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen.

Besonders bevorzugt enthält die erfindungsgemäße Kunststoffzusammensetzung als PVC-Typ Suspensions-, Masse-, Mikrosuspensions- oder Emulsions-PVC.

Bezogen auf 100 Massenteile Polymer enthalten die erfindungsgemäßen Kunststoffzusammensetzungen vorzugsweise von 5 bis 200, bevorzugt von 10 bis 150 Massenteile an Weichmacher. Das Massenverhältnis von Weichmacher zu Polymer beträgt 1:15 bis 15:1, vorzugsweise 1:5 bis 5:1.

Die erfindungsgemäßen Kunststoffzusammensetzungen können neben den genannten Bestandteilen weitere Bestandteile enthalten, die insbesondere ausgewählt sind aus der Gruppe bestehend aus Füllstoffen, Pigmenten, Thermostabilisatoren, Costabilisatoren, Antioxidantien, Viskositätsregulierer und Gleitmittel.

Die Thermostabilisatoren neutralisieren beispielsweise während und/oder nach der Verarbeitung von PVC abgespaltene Salzsäure und verhindern einen thermischen Abbau des Polymeren. Als Thermostabilisatoren kommen alle üblichen Stabilisatoren in fester und flüssiger Form in Betracht, beispielsweise auf Basis von Ca/Zn, Ba/Zn, Pb, Sn oder organischer Verbindungen (OBS), sowie auch säurebindende Schichtsilikate wie Hydrotalcit. Die erfindungsgemäßen Gemische können einen Gehalt von 0,5 bis 10, bevorzugt 1 bis 5, besonders bevorzugt von 1,5 bis 4 Massenteilen pro 100 Massenteilen Polymer an Thermostabilisator aufweisen.

Als sogenannte Costabilisatoren (d.h. Substanzen, die die Wirkung der Thermostabilisatoren verlängern, verbessern und/oder ergänzen), können z.B. Pflanzenölderivate wie z.B. epoxidiertes Sojabohnenöl oder eopxidiertes Leinöl eingesetzt werden.

Als Pigmente können im Rahmen der vorliegenden Erfindung sowohl anorganische als auch organische Pigmente eingesetzt werden. Der Gehalt an Pigmenten liegt zwischen 0,01 bis 10 Massen-%, bevorzugt 0,05 bis 5 Massen-%, besonders bevorzugt 0,1 bis 3 Massen-% pro 100 Massenteilen Polymer. Beispiele für anorganische Pigmente sind TiO₂, CdS, CoO/Al₂O₃, Cr₂O₃. Bekannte organische Pigmente sind beispielsweise Azofarbstoffe, Phthalocyaninpigmente, Dioxazinpigmente sowie Anilinpigmente.

Die erfindungsgemäßen Kunststoffzusammensetzungen können alle dem Stand der Technik entsprechenden Füllstoffe enthalten. Beispiele solcher Füllstoffe sind mineralische und/oder synthetische und/oder natürliche, organische und/oder anorganische Materialien, wie z. B. Calciumoxid, Magnesiumoxid, Calciumcarbonat, Bariumsulfat, Siliziumdioxid, Schichtsilikat, Industrieruß, Bitumen, Holz (z. B. pulverisiert, als Granulat, Mikrogranulat, Fasern usw.), Papier, Natur- und/oder Synthetikfasern usw.. Besonders bevorzugt handelt es sich bei mindestens einem der eingesetzten Füllstoffe um ein Calciumcarbonat oder ein Calcium-Magnesium-Carbonat.

Als viskositätssenkende Reagenzien können aliphatische oder aromatische Kohlenwasserstoffe aber auch Alkohole und/oder Carbonsäurederivate wie beispielsweise das als 2,2,4-Trimethyl-1,3-pentandiol-diisobutyrat oder Ester wie z.B. langkettige Benzoesäurealkylester eingesetzt werden. Viskositätssenkende Reagenzien werden den erfindungsgemäßen Zusammensetzungen insbesondere in Anteilen von 0,5 bis 50, bevorzugt 1 bis 30, besonders bevorzugt 2 bis 10 Massenteilen pro 100 Massenteilen Polymer zugegeben.

Ein weiterer Gegenstand der Erfindung sind Formkörper oder Folien hergestellt aus bzw. enthaltend die erfindungsgemäßen Kunststoffzusammensetzungen. Diese Formkörper oder Folien sind bevorzugt ein Fußbodenbelag, ein Wandbelag, ein Film / eine Folie, ein Schlauch, ein Profil, eine Dachbahn, eine Dichtungsbahn, eine Kabel- und Drahtummantelung, eine Plane, ein Werbebanner, Kunstleder, Verpackungsfolie, ein medizinischer Artikel, Spielzeug, eine Dichtung oder ein Einrichtungsgegenstand.

Die erfindungsgemäßen Diheptylester der Furandicarbonsäure besitzen in ihrer Verwendung als Weichmacher zahlreiche Vorteile gegenüber bekannten Weichmachern des Standes der Technik. So sind diese Verbindungen überraschenderweise im Gegensatz zu den homologen Di-n-butylfurandicarboxylaten und Di-n-hexylfurandicarboxylaten, welche beide als kristalliner Feststoff mit Schmelzpunkten weit oberhalb der Raumtemperatur vorliegen, gut zu verarbeitende (u.a. gut zu dosierende) Flüssigkeiten. Die Herstellung von flüssigen Zusammensetzungen, insbesondere Polymerzusammensetzungen wie Polymerpasten und Plastisolen im technischen Maßstab ist am besten mit flüssigen Weichmachern zu realisieren, da hier der Einsatz von Lösungsmitteln nicht notwendig ist. Lösemittel führen in der Regel auch zu einer "Verdünnung" des weichmachenden Effektes und müssen daher während der Herstellung wieder entfernt werden. Da dies in den seltensten Fällen quantitativ geschehen kann, sind hohe flüchtige (organische) Anteile sog. "VOC's" in den Halb- bzw. Fertigprodukten enthalten, die wiederum prohibitiv für den Einsatz solcher Produkte insbesondere im Innerraumbereich sowie in Fahrzeugen sind. Hinzu kommt die Gefahr des Auskristallisierens von festen Weichmachern im Halb- bzw. Fertigprodukt, was zu einer starken Verschlechterung der Materialeigenschaften, bis hin zum Materialversagen führt. Die alternativ zur Verwendung von Lösungsmitteln existierende Problemlösung, feste Weichmacher in geschmolzenem Zustand (d.h. bei erhöhter Temperatur) einzusetzen, ist in vielen Fällen technisch nicht umsetzbar. Auch besteht die Gefahr einer thermischen Schädigung von anderen Formulierungsbestandteilen.

Das thermische Verhalten (insbesondere die Schmelztemperatur und das Auftreten eines Glasübergangspunktes) der erfindungsgemäßen Furandicarbonsäurediheptylester hängt vom Verzweigungsgrad der Esterketten und der Zusammensetzung der Ester ab, und kann über die Zusammensetzung der zur Herstellung der Ester verwendeten Alkoholmischung gezielt eingestellt werden. Beim der Vermessung der erfindungsgemäßen Furandicarbonsäurediheptylester (Reinheit It. GC-Analyse mindestens 99 Flächen%) in einem Differentialkalorimeter (DSC) tritt nach Abkühlung auf -150 °C in der ersten Aufheizung kein Schmelzpunkt bei Temperaturen oberhalb von 20 °C auf, bevorzugt kein Schmelzpunkt bei Temperaturen oberhalb von 10 °C, besonders bevorzugt kein Schmelzpunkt bei Temperaturen oberhalb von 5 °C und insbesondere bevorzugt kein Schmelzpunkt bei Temperaturen oberhalb von 0 °C.

In einer besonderen Ausführungsform, insbesondere wenn gute Tieftemperatureigenschaften der Weichmacher bzw. der Kunststoffzusammensetzungen und Formkörper gewünscht werden, müssen mindestens 3 unterschiedliche Isomere in der zur Herstellung der Ester verwendeten Alkoholmischung vorhanden sein, bevorzugt mindesten 4, besonders bevorzugt mindestens 5 und insbesondere bevorzugt mindestens 6 Isomere. In diesem Fall wird bei der beschriebenen Vermessung mittels DSC eine Glasübergangstemperatur bei < 0 °C detektiert, bevorzugt bei < -10 °C, besonders bevorzugt bei < -20 °C und insbesondere bevorzugt bei < -30 °C.

Das rheologische Verhalten, insbesondere die Scherviskosität der erfindungsgemäßen Furandicarbonsäurediheptylester hängt vom Verzweigungsgrad der Esterketten und der Zusammensetzung der Ester ab, und kann ebenfalls über die Zusammensetzung der zur Herstellung der Ester verwendeten Alkoholmischung gezielt eingestellt werden. Die bei 20 °C bestimmte Scherviskosität der flüssigen erfindungsgemäßen Furandicarbonsäurediheptylester (Reinheit It. GC-Analyse mindestens 98 Flächen%) hängt vom Verzweigungsgrad der Esterketten und der Zusammensetzung der Ester ab. Sie liegt insbesondere bei maximal 120 mPa*s, bevorzugt bei maximal 110 mPa*s, besonders bevorzugt bei maximal 100 mPa*s und insbesondere bevorzugt bei unter 95 mPa*s.

Die Dichte der erfindungsgemäßen Ester hängt ebenfalls vom Verzweigungsgrad der Esterketten und der Zusammensetzung der Ester ab, und ist über die zur Esterherstellung verwendeten Alkohole einstellbar. Die bei 20 °C bestimmte Dichte der erfindungsgemäßen Ester (Reinheit It. GC-Analyse min. 99 FI.%) beträgt insbesondere maximal 1,1 g/cm³, bevorzugt maximal 1,08 g/cm³, besonders bevorzugt maximal 1,06 g/cm³ und insbesondere besonders bevorzugt maximal 1,05 g/cm³.

Ein weiterer Vorteil ist es, dass diese erfindungsgemäßen Heptylester ein hervorragendes Geliervermögen für Polymere, insbesondere für PVC aufweisen und überraschender Weise auch eine deutlich geringere Lösetemperatur für PVC besitzen als beispielsweise Di-n-butylfurandicarboxylat, obwohl sie eine deutlich längere Esterkette besitzen. Sie können somit schnell und bei niedrigen Temperaturen verarbeitet werden.

PVC-Plastisole / PVC-Pasten welche die erfindungsgemäßen Heptylester in einem Anteil von mindestens 10 ma% bezogen auf die insgesamt verwendeten Weichmacher enthalten, erreichen im mittels oszillierender Rheometrie durchgeführten Gelierversuch mit dynamischer Temperaturführung (konstante Heizrate) insbesondere eine Pastenviskosität von > 1.000 Pa*s bei Temperaturen von maximal 100 °C, bevorzugt von maximal 95 °C, besonders bevorzugt von maximal 90 °C, insbesondere bevorzugt von maximal 85 °C und insbesondere besonders bevorzugt von maximal 80 °C.

Die Temperatur bei der im oben beschriebenen Gelierversuch die maximale Viskosität erreicht wird, liegt insbesondere bei maximal 120 °C, bevorzugt bei maximal 115 °C, besonders bevorzugt bei maximal 110 °C, insbesondere bevorzugt bei maximal 105 °C und insbesondere besonders bevorzugt bei maximal 100 °C.

Es ist weiterhin festzustellen, dass PVC-Pasten auf Basis eines erfindungsgemäßen Diheptylesters der Furandicarbonsäure nur eine geringe Abhängigkeit der Pastenviskosität von der Schergeschwindigkeit aufweisen. Damit sind diese Weichmacher in einem weiten Schergeschwindigkeitsbereich und mit unterschiedlichsten Verarbeitungsmethoden verwendbar.

PVC-Pasten auf Basis der erfindungsgemäßen Heptylester weisen weiterhin auch eine deutlich geringere Geliertemperatur auf als beispielsweise Diisononylphthalatpasten. Diese können somit schneller und bei niedrigeren Temperaturen verarbeitet werden, so dass höherwertige Produkte entstehen.

Die erfindungsgemäßen Weichmacher weisen weitere Vorteile auf gegenüber den Weichmachern, welche Hexylester oder Butylester der Furandicarbonsäure enthalten. So wurde festgestellt, dass Prüfkörper mit dem erfindungsgemäßen Weichmacher eine deutlich bessere Beständigkeit bei erhöhter Temperatur aufweisen, weil sie geringere Masseverluste aufweisen. Auch wurde festgestellt, dass Proben, die den erfindungsgemäßen Weichmacher enthalten, eine deutlich bessere Thermostabilität aufweisen als die Vergleichsproben mit den Weichmachern des Standes der Technik.

Es wurde weiterhin auch überraschenderweise festgestellt, dass PVC-Folien enthaltend die erfindungsgemäßen Heptylester als Weichmacher eine sehr niedrige Opazität und damit eine hohe Transparenz aufweisen. Diese liegt zum Teil deutlich niedriger als bei Folien die auf Basis von Standardweichmachern hergestellt werden.

Insbesondere werden bei der Herstellung transparenter Folien (Foliendicke 0,9 - 1,1 mm) aus PVC-Plastisolen / PVC-Pasten welche die erfindungsgemäßen Heptylester in einem Anteil von mindestens 10 ma% bezogen auf die insgesamt verwendeten Weichmacher enthalten, Opazitätswerte von maximal 15 erzielt, bevorzugt maximal 14, besonders bevorzugt maximal 13, insbesondere bevorzugt maximal 12 und insbesondere besonders bevorzugt maximal 11. Der Gelbwert (Index YD 1925) der oben beschriebenen transparenten Folien liegt insbesondere bei maximal 15, bevorzugt bei maximal 14, besonders bevorzugt bei maximal 13, insbesondere bevorzugt bei maximal 12 und insbesondere besonders bevorzugt bei maximal 11.

Durch Abmischung mit Diisononylcyclohexancarbonsäureester (DINCH), welches eine deutlich schlechtere weichmachende Wirkung aufweist, wird ferner die Weichmachereffizienz der erfindungsgemäßen Heptylester der Furandicarbonsäure (DIHFDC) nur unwesentlich verschlechtert.

Prüfkörper welche das erfindungsgemäße DIHFDC enthalten zeigen bei Temperaturen > 0 °C eine deutlich höhere Flexibilität (d.h. einen niedrigeren Speichermodul) sowohl als Prüfkörper welche das lineare DNHFDC enthalten, als auch als Prüfkörper welche das einfach verzweigte und um ein C-Atom "längere" D2EHFDC enthalten. Die erfindungsgemäße Probe weist außerdem auch bei 0 °C und bei -30 °C eine ebenfalls deutlich höhere Flexibilität im Vergleich mit der D2EHFDC-Probe auf. Dies ist umso erstaunlicher, als die Glasübergangstemperatur der erfindungsgemäßen Probe im Vergleich mit den beiden anderen Proben höher liegt, und die Flexibilität von Weich-PVC-Prüfkörpern in der Regel mit abnehmender Glasübergangstemperatur steigt.

Die folgenden Beispiele sollen die Erfindung erläutern, ohne ihre Anwendungsbreite einzuschränken, die sich aus der Beschreibung und den Ansprüchen ergibt.

### BEISPIELE

### Beispiel 1

### Herstellung von Furan-2,5-dicarbonsäuredichlorid (II)

Die erfindungsgemäßen Ester wurden in einer zweistufigen Synthese ausgehend von Furan-2,5-dicarbonsäure über das Dichlorid hergestellt. In einem 250 mL-Dreihalskolben, mit Rückflusskühler und Tropftrichter wurden unter Argon 72,1 g (462 mmol) Furan-2,5-dicarbonsäure vorgelegt. In einem Zeitraum von 10 min wurden 165 g (1.39 mol) Thionylchlorid, versetzt mit einigen Tropfen N,N-Dimethylformamid, zugegeben. Die Suspension wurde auf Rückfluss-Temperatur erhitzt und das entstehende Gas durch Waschflaschen mit wässriger KOH-Lösung abgeleitet. Es wurde dann 4 h bis zur Beendigung der Gasentwicklung und vollständigen Auflösung des Feststoffes unter Rückfluss erhitzt. Die Isolierung des Produktes erfolgte, nach Abziehen von überschüssigem Thionylchlorid, durch destillative Aufreinigung (T = 110 °C, p = 0,0012 MPa). Hierbei resultierten 79,4 g Dichlorid als farbloser, kristalliner Feststoff (Ausbeute 89 %) mit einem Schmelzpunkt: von 79,5 - 80,0 °C. Furan-2,5-dicarbonsäuredichlorid wurde bis zur Weiterverwendung unter Schutzgas (Argon) im Dunklen bei Raumtemperatur gelagert.

**Herstellung der Furan-2,5-dicarbonsäureester aus Furan-2,5-dicarbonsäuredichlorid (II)** Unter Argon wurde in einem Dreihalskolben mit Rückflusskühler und Tropftrichter das Dichlorid vorgelegt und durch Erhitzen geschmolzen. Zur Flüssigkeit wurden 2,4 Äquivalente iso-Heptanol (Isomerengemisch; Exxal 7; Fa. Exxon Mobil) langsam zugetropft, wobei es zur exothermen Reaktion mit Gasentwicklung kam. Das entstehende Gas wurde durch Waschflaschen mit wässriger KOH-Lösung geleitet. Nach vollständiger Zugabe wurde 16 h bei einer Temperatur von 80 - 100 °C gerührt.

Der überschüssige Alkohol wurde in Gegenwart von Siedesteinen unter reduziertem Druck entfernt und das Rohprodukt destillativ aufgereinigt. Man erhält Diheptylfuran-2,5-dicarboxylat, das für die weiteren Versuche eingesetzt wird.

### Charakterisierung des Diheptylfuran-2,5-dicarboxylates

### 1.1 Bestimmung der Ester-Reinheit mittels gaschromatographischer Analyse (GC)

Die Bestimmung der Reinheit der hergestellten Ester mittels GC erfolgt mit einem GC-Automaten "6890N" von Agilent Technologies unter Verwendung einer DB-5-Säule (Länge: 20m, Innendurchmesser: 0,25 mm, Filmdicke 0,25 µm) von J&W Scientific und einem Flammenionisationsdetektor bei folgenden Rahmenbedingungen:

| | |
|---|---|
| Starttemperatur Ofen: 150 °C | Endtemperatur Ofen: 350 °C |
| (1) Heizrate 150-300 °C: 10 K/min | (2) Isotherm : 10 min. bei 300 °C |
| (3) Heizrate 300-350 °C: 25 K/min. | |
| Gesamtlaufzeit: 27 min. | |

Eingangstemperatur Einspritzblock: 300 °C Splittverhältnis: 200:1

| | |
|---|---|
| Splitfluss: 512,2 ml/min | Gesamtfluß: 517,7 ml/min. |
| Trägergas: Helium | Injektionsvolumen: 3 Mikroliter |
| | |
| Detektortemperatur: 350 °C | Brenngas: Wasserstoff |
| Flußrate Wasserstoff: 40 ml/min. | Flußrate Luft: 440 ml/min. |
| Makeup-Gas: Helium | Flußrate Makeup-Gas: 45 ml/min. |

Die Auswertung der erhaltenen Gaschromatogramme erfolgt manuell gegen vorhandene Vergleichssubstanzen die Angabe der Reinheit erfolgt in Flächenprozent. Auf Grund der hohen Endgehalte an Zielsubstanz von > 98 % ist der zu erwartende Fehler durch fehlende Kalibrierung auf die jeweilige Probensubstanz gering.

Die Messung ergab eine Reinheit des unter Beispiel 1 hergestellten Esters von 99,3 Flächen%.

### 1.2 Bestimmung der Dichte des hergestellten Esters

Die Bestimmung der Dichte der hergestellten Ester erfolgt mittels Biegeschwinger gemäß DIN 51757 - Verfahren 4.

Die Messung ergab eine Dichte von 1,0075 g/cm³.

### 1.3 Bestimmung der APHA-Farbzahl des hergestellten Esters

Die Bestimmung der Farbzahl der hergestellten Ester erfolgte gemäß DIN EN ISO 6271-2.

Die Messung ergab eine APHA-Farbzahl von 8.

### 1.4 Bestimmung der Säurezahl des hergestellten Esters

Die Bestimmung der Säurezahl der hergestellten Ester erfolgte gemäß DIN EN ISO 2114.

Die Bestimmung ergab eine Säurezahl von 0,012 mg KOH/g.

### 1.5 Bestimmung des Wassergehaltes des hergestellten Esters

Die Bestimmung des Wassergehaltes der hergestellten Ester erfolgte gemäß DIN 51777 Teil 1 (Direktes Verfahren).

Die Bestimmung ergab einen Wassergehalt von 0,023 %.

### 1.6 Bestimmung der Eigenviskosität des hergestellten Esters

Die Bestimmung der Eigenviskosität (Scherviskosität) des hergestellten Esters erfolgte unter Verwendung eines Physia MCR 101 (Fa. Anton-Paar) mit Z3-Meßsystem (DIN 25mm) im Rotationsmodus über folgendes Verfahren:
Ester und Meßsystem wurden zunächst auf eine Temperatur von 20°C temperiert, anschließend wurden folgende Punkte angesteuert:
   1. Eine Vorscherung von 100 s⁻¹ für den Zeitraum von 60 s, bei der keine Messwerte aufgenommen wurden (zur Nivellierung eventuell auftretender thixotroper Effekte und zur besseren Temperaturverteilung).
   2. Eine Frequenz-Abwärtsrampe, beginnend bei 500 s⁻¹ und endend bei 10 s⁻¹, aufgeteilt in eine logarithmische Reihe mit 20 Schritten mit jeweils 5 s Messpunktdauer (Verifizierung des newtonschen Verhaltens).

Der Ester zeigte ein newtonsches Fließverhalten.

Die Messung ergab eine Scherviskosität (bei 42 s⁻¹) von 90 mPa*s.

### 1.7 Bestimmung von Glasübergangstemperatur und Schmelztemperatur des hergestellten Esters mittels DSC

Die Bestimmung der und der Glasübergangstemperatur und der Schmelztemperatur erfolgt über Differentialkalorimetrie (DSC) gemäß DIN 51007 (Temperaturbereich von - 150 °C bis + 200 °C) aus der ersten Aufheizkurve bei einer Heizrate von 10K/min. Der Wendepunkt der Wärmestromkurve wird als Glasübergangstemperatur ausgewertet.

Die Messung ergab eine Glasübergangstemperatur von - 45 °C und eine Schmelztemperatur von - 19 °C, die Probe war bei zuvor 7 Tage bei Raumtemperatur flüssig gelagert worden.

### Beispiel 2

### Lösetemperatur der Weichmacher

Die Lösetemperatur gibt an, ab welcher Temperatur ein in einem kontinuierlich aufgeheizten Weichmacherüberschuss (96 g Weichmacher 4 g Polymer) dispergiertes PVC-Pulver gelöst wird, und lässt Rückschlüsse auf das Gelierverhalten zu. Di-(2-ethylhexyl)-furan-2,5-dicarboxylat (D2EHFDC), Di-n-hexylfuran-2,5-dicarboxylat (DNHFDC) und Di-n-butylfuran-2,5-dicarboxylat (DNBFDC) wurden unter Verwendung von 2-Ethylhexanol bzw. n-Hexanol analog zu Beispiel 1 hergestellt. Di-n-butylfuran-2,5-dicarboxylat und Di-n-hexylfuran-2,5-dicarboxylat (DNHFDC) wurde zunächst bei 50 °C aufgeschmolzen, Di-(2-ethylhexyl)-furan-2,5-dicarboxylat (D2EHFDC) und das erfindungsgemäße Di-iso-heptylfuran-2,5-dicarboxylat (DIHFDC) wurde auf 50 °C temperiert bevor das PVC-Pulver in der Flüssigkeit dispergiert und die Temperatur erhöht wurde.

### Versuchsdurchführung:

In einem 150 ml Becherglas werden 96 g des entsprechenden Weichmachers und 4 g des PVC's Lacovyl PB 1704 H (Fa. Arkema) eingewogen. Zu der Mischung werden ein Magnetrührfisch und ein an einem Stativ befestigtes Innenthermometer (Bereich: 0 °C - 250 °C, Anzeigegenauigkeit: 0,5 °C) gegeben. Mit einem Draht oder Klebeband wird an der Rückseite des Glases ein Papierstreifen, der den Schriftzug "Lösetemperatur" in der Schriftart "Times New Roman", Schriftgröße 12, so befestigt, dass der Schriftzug durch das Becherglas hindurch zu sehen ist. Danach wird die Heizplatte eines beheizbaren Laborrührgeräts (MR-Hei-Standard) auf 200 °C und die Drehzahl auf 600 U/min eingestellt. Nach Erreichen einer Innentemperatur der Flüssigkeit von 140 °C wurde die Solltemperatur nochmals auf 250 °C hochgeregelt. Die Lösetemperatur ist dann erreicht, wenn der Schriftzug durch die Flüssigkeit gerade klar lesbar geworden ist

Für die drei genannten Weichmacher wurden folgende Werte ermittelt (Doppelbestimmung), die in Tabelle 1 dargestellt sind:

**Tabelle 1: Ergebnisse der Versuche zur Lösetemperatur.**

| Weichmacher | Lösetemperatur [°C] |
|---|---|
| DNBFDC | 117 |
| DNHFDC | 104 |
| DIHFDC* | 112 |
| D2EHFDC | 120 |

| | |
|---|---|
| * erfindungsgemäß | |

Die Reihenfolge der Lösetemperaturen steigt folgerichtig mit steigender Esterkettenlänge an. Im Vergleich zum als Feststoff vorliegenden DNHFDC lässt sich das erfindungsgemäße DIHFDC allerdings viel besser verarbeiten wobei nur eine moderate Erhöhung der Lösetemperatur gegenüber dem DNHFDC zu beobachten ist.

### Beispiel 3 Verwendung der erfindungsgemäßen Furandicarbonsäurediheptylester allein und in Abmischung mit einem weiteren Weichmacher in einem PVC Plastisol zur Herstellung von Deckstrichfilmen für Fußbodenbeläge: Herstellung der Plastisole

Die mit den erfindungsgemäßen Weichmachern erzielbaren vorteilhaften Eigenschaften sollen im Folgenden an Plastisolen/Pasten aufgezeigt werden, wie sie beispielsweise zur Herstellung einer transparenten Deckschicht (sog. "Deckstrich") in mehrschichtig aufgebauten Fußbodenbelägen verwendet werden Die verwendeten Einwaagen in Gramm der Komponenten für die verschiedenen Pasten sind der nachfolgenden Tabelle 2 zu entnehmen. Beispiel 4 und 6 sind erfindungsgemäß, die anderen Beispiele 1, 2, 3, 5, 7 und 8 sind Vergleichsbeispiele.

**Tabelle 2: Rezepturen**

| Rezeptur | 1 | 2 | 3 | 4* | 5 | 6* | 7 |
|---|---|---|---|---|---|---|---|
| Vestolit B 7021 -- Ultra | 100 | 100 | 100 | 100 | 100 | 100 | 100 |
| Vestinol ® 9 | 50 | | | | | | |
| Hexamoll ® DINCH | | 50 | 10 | 10 | 10 | | |
| DNHFDC | | | 40 | | | | |
| DIHFDC | | | | 40 | | 50 | |
| D2EHFDC | | | | | 40 | | 50 |
| Drapex 39 | 3 | 3 | 3 | 3 | 3 | 3 | 3 |
| Mark CZ 149 | 2 | 2 | 2 | 2 | 2 | 2 | 2 |

| | | | | | | | |
|---|---|---|---|---|---|---|---|
| * = erfindungsgemäß | | | | | | | |

Die verwendeten Substanzen werden im Folgenden beschrieben:
Vestolit B 7021 - Ultra Mikrosuspensions-PVC (Homopolymer) mit einem K-Wert (bestimmt gemäß DIN EN ISO 1628-2) von 70; Fa. Vestolit GmbH.

Vestinol® 9 Diisononyl(ortho)phthalat (DINP), Weichmacher; Fa. Evonik Oxeno GmbH. Hexamoll ® DINCH : Diisononylcyclohexancarbonsäureester; Fa.BASF SE
DNHFDC: Di-n-hexylfuran-2,5-dicarboxylat (Hergestellt mit n- Hexanol analog Beispiel 1)
DIHFDC: Erfindungsgemäßer Furandicarbonsäuredi-iso-heptylester; gemäß Bsp. 1
D2EHFDC: Di-2ethylhexyl-furan-2,5-dicarboxylat (Hergestellt mit 2-Ethylhexanol analog Beispiel 1)
Drapex 39 Epoxidiertes Sojabohnenöl; Costabilisator mit weichmachender Wirkung; Fa. Galata Chemicals)
Mark CZ 149: Ca/Zn-Stabilisator, Fa. Galata Chemicals

Die flüssigen Bestandteile wurden vor den festen Bestandteilen in einem PE-Becher eingewogen. Die Mischung wurde mit einem Salbenspatel so eingerührt, dass kein unbenetztes Pulver mehr vorhanden war. Der Mischbecher wurde dann in die Klemmvorrichtung eines Dissolverrührers eingespannt. Mit einer Mischerscheibe wurde die Probe homogenisiert. Dabei wurde die Drehzahl von 330 U/min bis 2000 U/min erhöht, und so lange gerührt, bis die Temperatur an der Digitalanzeige des Thermofühlers 30,0 °C erreichte. Damit war sicher gestellt, dass die Homogenisierung der Paste bei einem definierten Energieeintrag erreicht wurde. Danach wurde die Paste sofort bei 25,0 °C temperiert.

Die Rezeptur 3 ließ sich nur durch Erwärmen des als Feststoff vorliegenden Furandicarboxylats herstellen. Im Anschluss an die Herstellung verfestigte sich die Paste jedoch so stark, dass keine weitere Verarbeitung durchgeführt werden konnte. Auf einen weiteren Vergleichsversuch mit reinem DNHFDC wurde darauf hin verzichtet.

Paste 4 zeigt die Wirkungsweise des erfindungsgemäßen Di-heptyl-furandicarboxylats in Abmischungen mit dem Weichmacher Hexamoll® DINCH. Paste 6 zeigt die Wirkungsweise des erfindungsgemäßen Di-heptyl-furandicarboxylats beim Einsatz als alleiniger Weichmacher.

### Beispiel 4 Verwendung der erfindungsgemäßen Furandicarbonsäurediheptylester in einem PVC Plastisol zur Herstellung von Deckstrichfilmen für Fußbodenbeläge: Messung der Pastenviskosität

Die Messung der Viskositäten der in Beispiel 3 hergestellten Pasten wurden mit einem Rheometer Physica MCR 101 (Fa. Anton Paar), welches über die zugehörige Software Rheoplus gesteuert wurde, wie folgt durchgeführt.

Die nach der Herstellung für 24 h bei 25 °C gelagerte Paste wurde im Vorratsbehälter nochmals mit einem Spatel homogenisiert und in dem Messsystem Z3 (Durchmesser 25 mm) gemäß Bedienungsanleitung vermessen. Die Messung wurde isotherm bei 25 °C durchgeführt. Folgende Punkte wurden angesteuert:
Eine Vorscherung von 100 s⁻¹ für den Zeitraum von 60 s, bei der keine Messwerte aufgenommen wurden (Nivellierung thixotroper Effekte).

Eine isotherme Abwärtsrampe, beginnend bei einer Schergeschwindigkeit von 200 s⁻¹ bis herunter zu 0,1 s⁻¹, aufgeteilt in eine logarithmische Reihe mit 30 Schritten mit jeweils 5 s Messpunktdauer. Die Ergebnisse der Messung sind in Tabelle 3 dargestellt.

**Tabelle 3: Ergebnisse der Viskositätsmessung (Schergeschwindigkeitsprofil)**

| Plastisolrezeptur gemäß Bsp. 3 | 1 | 2 | 3 | 4* | 5 | 6* | 7 |
|---|---|---|---|---|---|---|---|
| Scherviskosität bei Schergeschwindigkeit= 100/s [Pa*s] | 6 | 3,5 | 17,3 | 7,7 | 7,8 | 9,5 | 9,7 |
| Scherviskosität bei Schergeschwindigkeit= 10/s [Pa*s] | 3 | 1,7 | 8 | 4,1 | 4,2 | 5,1 | 5,2 |
| Scherviskosität bei Schergeschwindigkeit= 1/s [Pa*s] | 3 | 1,6 | 7,5 | 4,1 | 3,9 | 4,9 | 4,8 |
| Scherviskosität bei Schergeschwindigkeit= 0,1/s [Pa*s] | 4,2 | 2 | 10,3 | 5,6 | 5,7 | 5,8 | 6,5 |
| Schwankungsbreite [%] (Max. Visko - Min. Visko / Min. Visko)*100 | 100 | 119 | 131 | 88 | 100 | 98 | 102 |

| | | | | | | | |
|---|---|---|---|---|---|---|---|
| *= erfindungsgemäß | | | | | | | |

Im Vergleich der Pasten, die nur eine Weichmachersubstanz enthalten (Pasten 1, 2, 6, 7) weist die erfindungsgemäße Paste 6 die geringste Schwankungsbreite der Pastenviskosität auf. Mit reinem DNHFDC (Feststoff) lässt sich keine PVC-Paste herstellen, auch die DNHFDC-Mischung mit DINCH zeigt eine sehr hohe Pastenviskosität, die mit dem verwendeten Meßsystem bei hohen Schergeschwindigkeiten kaum noch sinnvoll bestimmt werden kann. Eine Abmischung des Weichmachers Hexamoll® DINCH mit dem erfindungsgemäßen Furandicarbonsäurediheptylester (Paste 4) führt neben einer deutlichen Reduktion des Viskositätsniveaus der Paste auch zu einer sehr starken Reduktion der Schwankungsbreite im Vergleich mit einer Paste welche allein Hexamoll® DINCH als Weichmacher enthält (Paste 2). Das generell erhöhte Viskositätsniveau der erfindungsgemäßen Pasten kann vom Fachmann leicht, z.B. durch Zusatz von Viskositätsadditiven oder Lösungsmitteln an die bei der jeweiligen Verarbeitungsart vorliegenden Gegebenheiten angepasst werden.

Weichmacher, mit herausragenden Geliereigenschaften, insbesondere sog. Schnellgelierer zeigen häufig eine mangelhafte Viskositätsstabilität der mit Ihnen hergestellten PVC-Pasten. Die Lagerstabilität von PVC-Pasten ist insofern von großer Bedeutung, als die Pasten im industriellen Maßstab in der Regel an einer zentralen Stellen hergestellt und dann nach und nach auf die Produktionsanlagen (z.B. Beschichtungsanlagen) verteilt werden. Zwischen Herstellung der Paste und deren Verarbeitung können dabei Zeiträume von bis zu 7 Tagen vergehen.

Um die Lagerstabilität der Pasten zu bestimmen wurden Viskositätsmessungen bei einer Schergeschwindigkeit von 100 s⁻¹ nach 2 h Lagerdauer, nach 24 h Lagerdauer und nach 7 Tagen Lagerdauer durchgeführt. Zwischen den Messungen wurden die Pasten im geschlossenen Behälter bei 25 °C gelagert. Die Durchführung der Messungen erfolgte wie oben beschrieben. Die Ergebnisse sind in Tabelle 4 zusammengestellt.

**Tabelle 4: Ergebnisse der Viskositätsmessung (Lagerstabilität)**

| Plastisolrezeptur gemäß Bsp. 3 | 1 | 2 | 3 | 4* | 5 | 6* | 7 |
|---|---|---|---|---|---|---|---|
| Scherviskosität bei Schergeschwindigkeit= 100/s 2 Stunden nach Herstellung der Plastisole [Pa*s] | 6 | 4,1 | 4,9 | 6,9 | 7,2 | 8,5 | 9 |
| Scherviskosität bei Schergeschwindigkeit= 100/s 24 Stunden nach Herstellung der Plastisole [Pa*s] | 6 | 3,5 | 17,3 | 7,7 | 7,8 | 9,5 | 9,7 |
| Scherviskosität bei Schergeschwindigkeit= 100/s 7 Tage nach Herstellung der Plastisole [Pa*s] | 6,4 | 3,5 | 18,6 | 8,4 | 8,3 | 10,9 | 10,8 |
| Schwankungsbreite [%] (Visko 7d -Visko 2h / Visko 2h)*100 | +7 | - 15 | + 280 | + 22 | + 15 | + 28 | + 20 |

| | | | | | | | |
|---|---|---|---|---|---|---|---|
| *= erfindungsgemäß | | | | | | | |

Die Ergebnisse zeigen, dass die Anforderung der Lagerstabilität von den erfindungsgemäßen Furandicarbonsäurediheptylestern sowohl als Einzelsubstanz (Probe 6) als auch in Mischung mit DINCH (Probe 4) erfüllt wird. Dagegen verfestigt sich die DNHFDC / DINCH-Mischung (Probe 3) mit zunehmender Lagerung so stark, dass sie nicht mehr verarbeitet werden kann.

### Beispiel 5 Verwendung der erfindungsgemäßen Furandicarbonsäurediheptylester in einem PVC Plastisol zur Herstellung von Deckstrichfilmen für Fußbodenbeläge: Bestimmung des Gelierverhaltens (Geliergeschwindigkeit)

Die Untersuchung des Gelierverhaltens der Pasten wurde im Physica MCR 101 im Oszillationsmodus mit einem Platte-Platte Meßsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| | Start-Temperatur: 25 °C |
| | End-Temperatur: 180 °C |
| | Heiz/Kühlrate: 5 °C/min |
| | Oszillations-Frequenz: 4-0,1 Hz Rampe logarithmisch |
| | Kreisfrequenz Omega: 10 1/s |
| | Anzahl Messpunkte: 63 |
| | Messpunktdauer: 0,5 min |
| | Automatische Spaltnachführung F : 0 N |
| | Konstante Messpunktdauer |
| | Spaltweite 0,5 mm |

Durchführung der Messung:
Auf die untere Messsystemplatte wurde mit dem Spatel ein Tropfen der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als ca. 6 mm rundum). Anschließend wurde die Temperierhaube über der Probe positioniert und die Messung gestartet. Bestimmt wurde die sog. komplexe Viskosität der Paste in Abhängigkeit von der Temperatur. Da eine bestimmte Temperatur in einer (durch die Heizrate von 5 °C/min. festgelegte) Zeitspanne erreicht wird, wird neben der Geliertemperatur auch eine Aussage zur Geliergeschwindigkeit des vermessenen Systems erhalten. Ein Einsetzen des Geliervorganges war in einem plötzlichen starken Anstieg der komplexen Viskosität zu erkennen. Je früher dieser Viskositätsanstieg einsetzt, desto besser ist die Gelierfähigkeit des Systems. Aus den erhaltenen Meßkurven wurden durch Interpolation für jedes Plastisol die Temperaturen bestimmt, bei der eine komplexe Viskosität von 1000 Pa ∗ s bzw. 10.000 Pa*s erreicht war. Zusätzlich wurde mittels Tangentenmethode die im vorliegenden Versuchsaufbau maximal erreichte Plastisolviskosität bestimmt, sowie durch Fällen eines Lotes die Temperatur, ab der die maximale Plastisolviskosität auftritt. Die Ergebnisse sind in Tabelle 5 zusammengestellt.

**Tabelle 5: Ergebnisse der Gelierversuche**

| Plastisolrezeptur | | | | | | | |
|---|---|---|---|---|---|---|---|
| (gemäß Bsp. 3) | 1 | 2 | 3 | 4* | 5 | 6* | 7 |
| Erreichen einer Plastisolviskosität von 1.000 Pa*s bei [°C] | 87 | 114 | 71 | 78 | 81 | 76 | 79 |
| Erreichen einer Plastisolviskosität von 10.000 Pa*s bei [°C] | 96 | 134 | 74 | 82 | 86 | 79 | 83 |
| Maximale Plastisolviskosität [Pa*s] | 27.900 | 19.500 | 67.800 | 48.000 | 31.400 | 62.000 | 46.800 |
| Temperatur beim Erreichen der maximalen Plastisolviskosität [°C] | 136 | 147 | 86 | 91 | 96 | 88 | 92 |

| | | | | | | | |
|---|---|---|---|---|---|---|---|
| *= erfindungsgemäß | | | | | | | |

Im Vergleich der Pasten, die nur eine Weichmachersubstanz enthalten (Pasten 1, 2, 6, 7) weist die erfindungsgemäße Paste die schnellste Gelierung auf. Die Gelierung verläuft auch deutlich schneller bzw. bei niedrigeren Temperaturen als die Gelierung der VESTINOL® 9-Paste. Auch die maximal erreichbaren Viskositäten während des Geliervorganges liegen für die erfindungsgemäße Probe eindeutig am höchsten. Analoges gilt für die Temperatur bei welcher die maximale Pastenviskosität während des Geliervorganges erreicht wird. Diese liegt für die erfindungsgemäße Probe am niedrigsten, wobei der Abstand zu den Proben mit Vestinol® 9 und DINCH besonders groß ist. Es werden mit den erfindungsgemäßen Furandicarbonsäurediheptylestern somit Weichmacher zur Verfügung gestellt, die über exzellente Geliereigenschaften verfügen und deren PVC-Pasten zu Formkörpern mit hoher Festigkeit ausgeliert werden können. Die die erfindungsgemäßen Weichmacher enthaltenden PVC-Zusammensetzungen können insbesondere mit hohen Geschwindigkeiten und bei niedrigen Verarbeitungstemperaturen verarbeitet werden. Die Zumischung eines relativ geringen Anteils an Hexamoll® DINCH, die zu einer signifikanten Reduktion der Pastenviskosität führt (siehe Bsp. 4) zeigt nur einen untergeordneten Einfluss auf die Geliereigenschaften. Auch mit DINCH wird immer noch eine deutlich schnellere Gelierung ermöglicht als dies mit dem Standardweichmacher VESTINOL® 9 der Fall ist.

### Beispiel 6: Verwendung der erfindungsgemäßen Furandicarbonsäurediheptylester in einem PVC Plastisol zur Herstellung von Deckstrichfilmen für Fußbodenbeläge: Bestimmung der Shore A Härte von Gießlingen (Weichmachereffizienz)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore-Härte. Da in der Praxis Formulierungen / Rezepturen häufig auf eine bestimmte Shore-Härte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet.

Zur Bestimmung der Shore-Härten wurden die gemäß Beispiel 3 hergestellten Pasten in runde Gießformen aus Messing mit einem Durchmesser von 42 mm gegossen (Einwaage: 20,0 g). Dann wurden die Pasten in den Formen im Umlufttrockenschrank 30 min bei 200 °C geliert, nach Abkühlung entnommen und vor der Messung mindestens 24 Stunden im Trockenschrank (25 °C) gelagert. Die Dicke der Scheiben betrug ca. 12 mm.

Die Härte-Messungen wurden nach DIN 53 505 mit einem Shore-A-Messgerät der Fa. Zwick-Roell durchgeführt, der Messwert jeweils nach 3 Sekunden abgelesen. An jedem Probekörper (z.B. Gießling) wurden Messungen an drei verschiedenen Stellen durchgeführt, und ein Mittelwert gebildet. Die Ergebnisse der Härtebestimmung sind in Tabelle 6 zusammengestellt.

**Tabelle 6: Ergebnisse der Shore A Messungen**

| Plastisolrezeptur | | | | | | | |
|---|---|---|---|---|---|---|---|
| (gemäß Bsp. 3) | 1 | 2 | 3 | 4* | 5 | 6* | 7 |
| Shore A | 82 | 84 | 75 | 78 | 80 | 77 | 79 |

| | | | | | | | |
|---|---|---|---|---|---|---|---|
| *= erfindungsgemäß | | | | | | | |

Im Vergleich der Pasten, die nur eine Weichmachersubstanz enthalten (Pasten 1, 2, 6, 7) weist der Gießling, welcher aus der erfindungsgemäßen Paste 6 hergestellt wurde, die niedrigste Shore A-Härte -also die größte "Weichheit" auf-. Es werden somit Ester zur Verfügung gestellt, die in PVC-Mischungen eine außerordentlich hohe Weichmachereffizienz besitzen, was zu einem deutlichen Einsparpotential hinsichtlich der benötigten Weichmachermenge im Vergleich z.B. mit dem Standardweichmacher Vestinol® 9 führt. Überraschender Weise wird auch durch Abmischung mit dem deutlich weniger effizienten DINCH (Probe 4) die Weichmachereffizienz der erfindungsgemäßen Ester nur geringfügig herabgesetzt und liegt immer noch weit besser als beim Standardweichmacher.

### Beispiel 7: Verwendung der erfindungsgemäßen Furandicarbonsäuredi-heptylester in einem PVC Plastisol zur Herstellung von Deckstrichfilmen für Fußbodenbeläge: Bestimmung von Transparenz und Gelbwert der transparenten Deckstrichfolien

Die Herstellung der Folien erfolgte nach einer Reifezeit der Plastisole von 24 Stunden (bei 25 °C). Für die Folienherstellung wurde am Rollrakel eines Mathis Labcoaters (Hersteller: Fa. W. Mathis AG) ein Rakelspalt von 1,40 mm eingestellt. Dieser wurde mit einer Fühlerblattlehre kontrolliert und ggf. nachgestellt. Die hergestellten Pasten wurden auf ein in einem Rahmen plan eingespanntes Hochglanzpapier (Ultracast Patent; Fa. Sappi Ltd.) mittels dem Rollrakel des Mathis Labcoaters aufgerakelt. Das aufgerakelte Plastisol wurde nun für 2 min im Mathis Ofen bei 200 °C ausgeliert. Nach Abkühlung wurde die Foliendicke mit Hilfe eines Dickenschnellmessers (KXL047; Fa. Mitutoyo) mit einer Genauigkeit von 0,01 mm bestimmt. Die Foliendicke dieser Folie betrug bei dem angegebenen Rakelspalt in allen Fällen zwischen 0,95 und 1,05 mm. Die Messung der Dicke wurde an drei unterschiedlichen Stellen der Folie durchgeführt.

Die Transparenz ist ein wesentliches Kriterium zur Qualitätsbeurteilung von PVC-Deckstrichen im Fußbodenbereich, da nur bei hoher Transparenz (= geringer Opazität) ein optimales Gesamterscheinungsbild erzielt werden kann. Die Transparenz einer PVC-Deckstrichfolie gilt auch als Maß für die Verträglichkeit der zur Folienherstellung verwendeten Rezepturbestandteile, insbesondere als Maß zur Bewertung der Verträglichkeit von PVC-Matrix und Weichmacher. Hohe Transparenz (= geringe Opazität) bedeutet in der Regel eine gute Verträglichkeit. Die Bestimmung der Opazität erfolgte mit einem "Spectro Guide" Gerät der Fa. Byk Gardner. Als Hintergrund für die Opazitätsmessungen wurden eine weiße Kachel und eine schwarze Kachel benutzt. Über das Menü am Farbmessgerät wurde die Opazitätsmessung angewählt. Die Messungen selbst wurden an 3 unterschiedlichen Stellen der Proben durchgeführt und automatisch ausgewertet (Durchschnittswert).

Der Gelbwert ist ein weiteres wichtiges Qualitätskriterium. Eine Gelbfärbung im Deckstrich kann zu einer erheblichen optischen Beeinträchtigung eines Fußbodendekors führen, weshalb beim PVC-Deckstrich in der Regel nur sehr geringe Gelbwerte toleriert werden können. Die Gelbverfärbung kann einerseits durch Rezepturbestandteile (wie auch durch ihre Neben- und Abbauprodukte) hervorgerufen werden, andererseits durch (z.B. thermooxidativen) Abbau während des Herstellprozesses und/oder während der Verwendung des Deckstriches bzw. des Fußbodenbelages auftreten.

Der Gelbwert (Index YD 1925) ist ein Maß für Gelbverfärbung eines Probekörpers. Die Farbmessung erfolgte mit einem "Spectro Guide" Gerät der Fa. Byk-Gardner. Als Hintergrund für die Farbmessungen wurde eine weiße Referenz-Kachel benutzt. Folgende Parameter wurden eingestellt:
Lichtart: C/2°
Anzahl Messungen: 3
Anzeige: CIE L*a*b*
Messindex: YD1925

Die Messungen selbst wurden an 3 unterschiedlichen Stellen der Proben (für Effekt- und Glattschäume bei einer Plastisol-Rakeldicke von 200 µm) durchgeführt. Die Werte aus den 3 Messungen wurden gemittelt. Tabelle 7 zeigt die Ergebnisse.

**Tabelle 7: Opazität und Gelbwert transparenter Deckstrichfolien**

| Plastisolrezeptur (gemäß Bsp. 3) | 1 | 2 | 3 | 4* | 5 | 6* | 7 |
|---|---|---|---|---|---|---|---|
| Opazität [-] | 10,2 | 9,9 | n.bb. | 9,9 | 9,8 | 10,1 | 9,9 |
| Gelbwert [-] | 9,0 | 9,0 | n.bb. | 8,5 | 9,6 | 9,0 | 10,3 |

| | | | | | | | |
|---|---|---|---|---|---|---|---|
| * = erfindungsgemäß; n.bb.= Nicht bestimmbar, Probe stark inhomogen. | | | | | | | |

Die erfindungsgemäßen Furandicarbonsäurediheptylester (Beispiele 4 und 6) zeigen sowohl als Einzelsubstanz als auch als Mischung mit DINCH eine hervorragende Verträglichkeit mit PVC ausgedrückt in sehr niedrigen Opazitätswerten. Zusätzlich werden ebenfalls sowohl als Einzelsubstanz als auch als Mischung mit DINCH sehr niedrige Gelbwerte erreicht. Dies ist insofern überraschend, als Weichmacher, welche auf Basis von nachwachsenden Rohstoffen, insbesondere von Zuckern hergestellt werden in der Regel höhere Gelbwerte aufweisen. Die Vergleichsprobe, welche DNHFDC enthält (Probe 3) ist hingegen inhomogen und nicht für die im vorliegenden Fall beabsichtigte Anwendung (transparenter Fußbodendeckstrich) geeignet.

### Beispiel 8: Verwendung der erfindungsgemäßen Furandicarbonsäurediheptylester in einem PVC Plastisol zur Herstellung von Deckstrichfilmen für Fußbodenbeläge: Wasserlagerung (bei 30 °C) und Warmlagerung (bei 80 °C) der Deckstrichfilme

Die Alterungsbeständigkeit unter verschiedenen Umgebungsbedingungen ist ein weiteres wesentliches Qualitätskriterium für PVC-Weichmacher. Insbesondere das Verhalten gegenüber Wasser (Wasseraufnahme & Auschwaschverhalten von Rezepturbestandteilen) und gegenüber erhöhten Temperaturen (Ausdunstung von Rezepturbestandteilen & thermische Alterung) bietet einen Einblick in die Alterungsbeständigkeit.

Nimmt ein Kunststoff-Fußboden in größerem Umfang Wasser auf, so verändern sich dadurch einerseits seine Materialeigenschaften, andererseits auch sein optisches Erscheinungsbild (z.B. Eintrübung). Eine hohe Wasseraufnahme ist demnach in der Regel unerwünscht. Das Auswaschverhalten ist ein zusätzliches Kriterium für die Permanenz der Formulierungsbestandteile unter Gebrauchsbedingungen. Dies gilt insbesondere für Stabilisatoren, Weichmacher und/oder ihre Bestandteile, da eine Konzentrationsminderung im Kunststoff-Fußboden bei diesen Rezepturbestandteilen sowohl die Materialeigenschaften verschlechtern, als auch die Lebensdauer des Fußbodenbelages dramatisch reduzieren kann.

Für die Bestimmung der Wasserbeständigkeit wurden ausgelierte 1 mm-Polymerfilme (Gelierbedingungen im Mathisofen: 200 °C/2 min.) verwendet. Als Prüfkörper wurden aus den Folien Kreise mit 3 cm Durchmesser ausgeschnitten. Vor der Wasserlagerung wurden die Prüfkörper für 24 Stunden in einem mit Trockenmittel (KC-Trockenperlen, Fa. BASF SE) ausgerüsteten Exsikkator bei 25 °C gelagert. Das Ausgangsgewicht (Einwage) wurde mit einer Analysenwaage auf 0,1 mg genau bestimmt. Die Prüfkörper wurden nun in einem mit VE-Wasser gefülltem Schüttelbad (Typ "WNB 22" mit Peltierkühlvorrichtung "CDP"; Fa. Memmert GmbH) bei einer Temperatur von 30 °C für 7 Tage mit Probenhaltern unter der Wasseroberfläche gelagert und kontinuierlich bewegt. Nach der Lagerung wurden die Kreise dem Wasserbad entnommen, abgetrocknet und ausgewogen (= Gewicht nach 7 Tagen). Aus der Differenz zur Einwage wurde die Wasseraufnahme berechnet. Nach der Auswage wurden die Prüfkörper wiederum für 24 Stunden in einem mit Trockenmittel (KC-Trockenperlen) ausgerüsteten Exsikkator bei 25 °C gelagert und anschließend nochmals ausgewogen (Endauswaage = Gewicht nach Trocknung). Aus der Differenz zur Einwage wurde der Massenverlust durch Wasserlagerung (= Verlust durch Auswaschungen) berechnet.

Für die Bestimmung der Beständigkeit gegenüber thermischer Alterung wurden wie oben beschrieben Prüfkörper hergestellt. Die Prüfkörper wurden wie oben beschrieben vorgetrocknet und Eingewogen, außerdem wurde wie in Bsp. 7 beschrieben der Gelbwert bestimmt. Anschließend wurden die Prüfkörper im Trockenschrank bei 80 °C eine Woche gelagert. Es wurde danach der Massenverlust der Prüfkörper und erneut ihre Gelbwert ermittelt. Die Ergebnisse sind in Tabelle 8 zusammengestellt.

**Tabelle 8: Ergebnisse der Untersuchungen zur Alterungsbeständigkeit**

| | | | | | | | |
|---|---|---|---|---|---|---|---|
| Plastisolrezeptur (gemäß Bsp. 3) | 1 | 2 | 3 | 4* | 5 | 6* | 7 |
| Wasseraufnahme nach 7 Tagen bei 30 °C [ma%] | 1,1 | 1,2 | 1,2 | 1,2 | 1,1 | 1,2 | 1,1 |
| Auswaschverlust nach 7 Tagen bei 30 °C [ma%] | 0 | 0 | 0,1 | 0 | 0 | 0 | 0 |
| Massenverlust nach 7 Tagen bei 80°C [ma%] | 0 | 0,1 | 3,4 | 1,5 | 0,6 | 1,6 | 0,7 |
| Gelbwert vor Warmlagerung | 9,0 | 9,0 | n.bb. | 8,5 | 9,6 | 9,0 | 10,3 |
| Gelbwert nach 7 Tagen bei 80°C | 20,7 | 23,3 | n.bb. | 20,5 | 22,9 | 21 | 24,2 |

| | | | | | | | |
|---|---|---|---|---|---|---|---|
| *= erfindungsgemäß; n.bb.: nicht bestimmbar, Probe stark inhomogen | | | | | | | |

Die Wasseraufnahme aller Proben liegt auf gleichem Niveau wie bei dem Standardweichmacher Vestinol® 9. Auswaschungen sind in keinem Fall feststellbar. Die Wasserbeständigkeit der erfindungsgemäßen Furandicarbonsäurediheptylester ist somit als ausgezeichnet zu bewerten.

Bei der Warmlagerung bei 80 °C zeigen sich hingegen starke Unterschiede zwischen den Proben. Überraschenderweise zeigt der erfindungsgemäße Prüfkörper 4, einen deutlich geringeren Masseverluste als der Prüfkörper (3), welcher das DNHFDC (Estergruppen mit 6 Kohlenstoffatomen) enthält. Prüfkörper mit dem erfindungsgemäßen Weichmacher weisen somit eine deutlich bessere Beständigkeit bei erhöhter Temperatur auf. Bezüglich der Verfärbung zeigt im Vergleich der Proben, welche nur eine Weichmachersubstanz enthalten (1, 2, 6, 7) überraschenderweise die Probe welche die erfindungsgemäßen Furandicarbonsäurediheptylester enthält nur eine geringe Gelbverfärbung in Folge der Temperaturbelastung und liegt damit auf gleichem Niveau wie die Probe welche den Standardweichmacher Vestinol® 9 enthält, sowie überraschender Weise deutlich besser als die DINCH-Probe. Dies ist insofern erstaunlich, weil Weichmacher welche ganz oder teilweise auf nachwachsenden Rohstoffen basieren (insbesondere wenn es sich um Zucker handelt) in der Regel anfälliger für Vergilbung bei höheren Temperaturen sind.

Es werden somit Ester zur Verfügung gestellt, die in ausgelierten PVC-Filmen zu einer außerordentlich guten Beständigkeit gegenüber Wasser, und einer ausgezeichnete Thermostabilität führen.

### Beispiel 9: Verwendung der erfindungsgemäßen Furandicarbonsäurediheptylester in einem PVC Plastisol zur Herstellung von Deckstrichfilmen für Fußbodenbeläge: Bestimmung der Flexibilität (DMTA)

An Prüfkörpern, die aus ausgelierten Folien (Folienherstellung gemäß Bsp. 7 im Mathisofen bei 200 °C /2 min.) hergestellt wurden, wurden DMTA-Messungen gemäß DIN 65583 durchgeführt. Für die dynamisch-mechanisch-thermische Analyse (DMTA) werden durch Anwendung einer oszillierenden Kraft auf einen Probenkörper frequenzabhängig und temperaturabhängig die viskoelastischen Eigenschaften erfasst, und die elastischen Modul- und Dämpfungswerte ermittelt. Das Maximum des Verlustmoduls wird dabei als Glasübergangstemperatur ausgewertet. Die nachfolgende Tabelle 9 zeigt die Ergebnisse.

**Tabelle 9: Ergebnisse der DMTA-Messungen**

| | | | | | | | |
|---|---|---|---|---|---|---|---|
| Plastisolrezeptur (gemäß Bsp. 3) | 1 | 2 | 3 | 4* | 5 | 6* | 7 |
| Glasübergangspunkt [°C] | -38 | -43 | -32 | -28 | - 30 | -24 | -26 |
| Speichermodul bei + 30 °C [M Pa] | 11 | 19 | 7 | 4 | 8 | 6 | 8 |
| Speichermodul bei 0 °C [MPa] | 92 | 116 | 59 | 97 | 101 | 109 | 114 |
| Speichermodul bei - 30 °C [M Pa] | 716 | 646 | 803 | 1036 | 1059 | 1221 | 1129 |

| | | | | | | | |
|---|---|---|---|---|---|---|---|
| *= erfindungsgemäß | | | | | | | |

Im Vergleich der Prüfkörper, die nur eine Weichmachersubstanz enthalten (Pasten 1, 2, 6, 7) weist der Prüfkörper, welcher aus der erfindungsgemäßen Paste 6 hergestellt wurde, überraschenderweise bei + 30 °C die höchste Flexibilität (d.h. den niedrigsten Speichermodul) auf. Betrachtet man die Abmischungen mit DINCH (Pasten 3, 4, 5) so ist hier bei + 30 °C für den Prüfkörper welcher die erfindungsgemäßen Furandicarbonsäurediheptylester enthält (Probe 4) sowohl eine höhere Flexibilität (d.h. ein niedrigerer Speichermodul) als beim Prüfkörper welcher das lineare DNHFDC (Probe 3) enthält, als auch beim Prüfkörper welcher das einfach verzweigte D2EHFDC (Probe 5) enthält zu erkennen, obwohl sie einen wesentlich höheren Verzweigungsgrad als D2EHFDC aufweisen. Die erfindungsgemäße Probe weist außerdem auch bei 0 °C und bei -30 °C eine ebenfalls deutlich höhere Flexibilität im Vergleich mit der D2EHFDC-Probe auf. Dies ist umso erstaunlicher, als die Glasübergangstemperatur der erfindungsgemäßen Probe im Vergleich mit den beiden anderen Proben höher liegt, und die Flexibilität von Weich-PVC-Prüfkörpern in der Regel mit abnehmender Glasübergangstemperatur steigt.

## Patentansprüche

1. Diheptylester der Furandicarbonsäure, **dadurch gekennzeichnet,**
**dass** er folgende Eigenschaften aufweist:
1) seine Eigenviskosität bei 25 °C beträgt maximal 120 mPa*s
2) beim Vermessen mit einem Differentialkalorimeter tritt kein Schmelzsignal bei Temperaturen > 20 °C auf.

2. Diheptylester nach Anspruch 1,
**dadurch gekennzeichnet**,
das seine Dichte bei 20 °C maximal 1,1 g/cm³ beträgt.

3. Weichmacherzusammensetzung,
welche mindestens einen Diheptylester gemäß einem der Ansprüche 1 oder 2 enthält, wobei
sie mindestens zwei isomere Diheptylfuran-2,5-dicarboxylate enthält.

4. Weichmacherzusammensetzung nach Anspruch 3, **dadurch gekennzeichnet, dass** die isomeren Diheptylfuran-2,5-dicarboxylate isomere Heptylgruppen enthalten.

5. Weichmacherzusammensetzung nach einem der Ansprüche 3 oder 4,
**dadurch gekennzeichnet,**
**dass** keiner der isomeren Diheptylfuran-2,5-dicarboxylate einen Anteil von mehr als 99,9 Gew.-% im Estergemisch besitzt.

6. Weichmacherzusammensetzung nach Anspruch 4,
**dadurch gekennzeichnet,**
**dass** mindestens eine der isomeren Heptylgruppen ausgewählt ist aus: n-Heptyl-, 2-Metylhexyl-, 3-Methylhexyl-, 4-Methylhexyl-, 5-Methylhexyl-, 2,2-Dimethylpentyl-, 2,3-Dimethylpentyl-, 3,3-Dimethylpentyl-, 2,4-Dimethylpentyl-, 3-Ethylpentyl-, 2,2,3-Trimethylbutyl und 2-Ethyl-3-methylbutyl-Gruppen.

7. Weichmacherzusammensetzung nach mindestens einem der Ansprüche 3 bis 6,
**dadurch gekennzeichnet,**
**dass** sie mindestens einen zusätzlichen Weichmacher ausgewählt aus der Gruppe Alkylbenzoate, Dialkyladipate, Glycerinester, Citronensäuretrialkylester, acylierte Citronensäuretrialkylester, Trialkylmellitate, Glykoldibenzoate, Dialkylterephthalate, Dialkylphthalate, Isosorbiddialkanoate, Dialkylester der 1,2-, 1,3- oder 1,4-Cyclohexandicarbonsäure enthält.

8. Verfahren zur Herstellung eines Diheptylesters nach Anspruch 1 oder 2 umfassend die Verfahrensschritte:
a) In Kontakt bringen von Furandicarbonsäure und/oder mindestens eines Furandicarbonsäurederivates, mit einem oder mehreren aliphatischen Alkoholen mit 7-Kohlenstoffatomen,
b) Erhitzen der beschriebenen Reaktionsmischung auf eine Temperatur > 50 °C und Veresterung bzw. Umesterung unter (bevorzugt thermischer) Abtrennung mindestens einer niedermolekularen Substanz aus dem Reaktionsgemisch.

9. Verfahren zur Herstellung eines Diheptylesters nach Anspruch 1 oder 2 umfassend die Verfahrensschritte:
a) In Kontakt bringen von 5-Hydroxymethylfurfural und / oder mindestens eines Furanderivates mit einem oder mehreren aliphatischen Alkoholen mit 7-Kohlenstoffatomen, sowie mindestens einem Katalysator und mindestens einer sauerstoffhaltigen Komponente,
b) Temperieren der beschriebenen Reaktionsmischung auf eine Temperatur > 0 °C und Durchführung einer oxidativen Veresterung.

10. Verwendung der Weichmacherzusammensetzung nach einem der Ansprüche 3 bis 7 als Weichmacher für Polymere.

11. Verwendung eines Diheptylesters nach Anspruch 1 oder 2, in Klebstoffen, Dichtungsmassen, Beschichtungsmassen, Lacken, Farben, Plastisolen, Pasten, Kunstleder, Fußbodenbeläge, Unterbodenschutz, Gewebebeschichtungen, Tapeten oder Tinten.

12. Kunststoffzusammensetzung enthaltend mindestens einen Diheptylester nach Anspruch 1 oder 2.

13. Kunststoffzusammensetzung nach Anspruch 12,
**dadurch gekennzeichnet,**
**dass** mindestens ein Polymer enthalten ist, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymeren der genannten Polymere oder deren monomeren Einheiten.

14. Kunststoffzusammensetzung nach Anspruch 12 oder 13,
**dadurch gekennzeichnet,**
**dass** Weichmacher in einer Menge von 5 bis 200 Massenteilen pro 100 Massenteile Polymer enthalten ist.

15. Kunststoffzusammensetzung nach einem der Ansprüche 12 bis 14,
**dadurch gekennzeichnet,**
**dass** das mindestens eines der enthaltenen Polymere ein Copolymer von Vinylchlorid mit einem oder mehreren Monomeren ausgewählt aus der Gruppe bestehend aus Vinylidenchlorid, Vinylacetat, Vinylpropionat, Vinylbutyrat, Vinylbenzoat, Methylacrylat, Ethylacrylat, oder Butylacrylat ist.

16. Formkörper oder Folie enthaltend eine Kunststoffzusammensetzung gemäß einem der Ansprüche 12 bis 15.

17. Formkörper oder Folie nach Anspruch 16,
wobei die Folie oder der Formkörper ein Fußbodenbelag, ein Wandbelag, ein Schlauch, ein Profil, ein Polymerfilm, eine Dachbahn, eine Dichtungsbahn, eine Kabel- oder Drahtummantelung, eine Plane, ein Werbebanner, Kunstleder, Verpackungsfolie, ein medizinischer Artikel, Spielzeug, eine Dichtung, ein Einrichtungsgegenstand ist.

## Claims

1. Diheptyl ester of furandicarboxylic acid, **characterized in that**
it has the following properties:
1) its intrinsic viscosity at 25°C is not more than 120 mPa*s
2) when analysed with a differential calorimeter, there is no melting signal at temperatures > 20°C.

2. Diheptyl ester according to Claim 1,
**characterized in that**
its density at 20°C is not more than 1.1 g/cm³.

3. Plasticizer composition
comprising at least one diheptyl ester according to either of Claims 1 and 2,
wherein
the composition comprises at least two isomeric diheptyl furan-2,5-dicarboxylates.

4. Plasticizer composition according to Claim 3,
**characterized in that** the isomeric diheptyl furan-2,5-dicarboxylates comprise isomeric heptyl groups.

5. Plasticizer composition according to either of Claims 3 and 4,
**characterized in that**
none of the isomeric diheptyl furan-2,5-dicarboxylates has a proportion of more than 99.9% by weight in the ester mixture.

6. Plasticizer composition according to Claim 4,
**characterized in that**
at least one of the isomeric heptyl groups is selected from the group of: n-heptyl, 2-methylhexyl, 3-methylhexyl, 4-methylhexyl, 5-methylhexyl, 2,2-dimethylpentyl, 2,3-dimethylpentyl, 3,3-dimethylpentyl, 2,4-dimethylpentyl, 3-ethylpentyl, 2,2,3-trimethylbutyl and 2-ethyl-3-methylbutyl groups.

7. Plasticizer composition according to at least one of Claims 3 to 6,
**characterized in that**
it comprises at least one additional plasticizer selected from the group of alkyl benzoates, dialkyl adipates, glyceryl esters, trialkyl citrates, acylated trialkyl citrates, trialkyl mellitates, glycol dibenzoates, dialkyl terephthalates, dialkyl phthalates, isosorbide dialkanoates, dialkyl esters of 1,2-, 1,3- or 1,4-cyclohexanedicarboxylic acid.

8. Process for preparing a diheptyl ester according to Claim 1 or 2, comprising the process steps of:
a) contacting furandicarboxylic acid and/or at least one furandicarboxylic acid derivative with one or more aliphatic alcohols having 7 carbon atoms,
b) heating the reaction mixture described to a temperature of > 50°C and esterifying or transesterifying while removing (preferably thermally) at least one low molecular weight substance from the reaction mixture.

9. Process for preparing a diheptyl ester according to Claim 1 or 2, comprising the process steps of:
a) contacting 5-hydroxymethylfurfural and/or at least one furan derivative with one or more aliphatic alcohols having 7 carbon atoms and at least one catalyst and at least one oxygen-containing component,
b) adjusting the reaction mixture described to a temperature of > 0°C and conducting an oxidative esterification.

10. Use of the plasticizer composition according to any of Claims 3 to 7 as a plasticizer for polymers.

11. Use of a diheptyl ester according to Claim 1 or 2 in adhesives, sealing compounds, coating materials, lacquers, paints, plastisols, pastes, synthetic leather, floor coverings, underbody protection, fabric coatings, wallpaper or inks.

12. Polymer composition comprising at least one diheptyl ester according to Claim 1 or 2.

13. Polymer composition according to Claim 12,
**characterized in that**
it comprises at least one polymer selected from polyvinyl chloride (PVC), polyvinylidene chloride (PVDC), polyacrylates, especially polymethyl methacrylate (PMMA), polyalkyl methacrylate (PAMA), fluoropolymers, especially polyvinylidene fluoride (PVDF), polytetrafluoroethylene (PTFE), polyvinyl acetate (PVAc), polyvinyl alcohol (PVA), polyvinyl acetals, especially polyvinyl butyral (PVB), polystyrene polymers, especially polystyrene (PS), expandable polystyrene (EPS), acrylonitrile-styrene-acrylate (ASA), styrene acrylonitrile (SAN), acrylonitrile-butadiene-styrene (ABS), styrene-maleic anhydride copolymer (SMA), styrene-methacrylic acid copolymer, polyolefins, especially polyethylene (PE) or polypropylene (PP), thermoplastic polyolefins (TPO), polyethylene-vinyl acetate (EVA), polycarbonates, polyethylene terephthalate (PET), polybutylene terephthalate (PBT), polyoxymethylene (POM), polyamide (PA), polyethylene glycol (PEG), polyurethane (PU),
thermoplastic polyurethane (TPU), polysulphides (PSu), biopolymers, especially polylactic acid (PLA), polyhydroxybutyral (PHB), polyhydroxyvaleric acid (PHV), polyesters, starch, cellulose and cellulose derivatives, especially nitrocellulose (NC), ethylcellulose (EC), cellulose acetate (CA), cellulose acetate/butyrate (CAB), rubber or silicones, and mixtures or copolymers of the polymers mentioned or monomeric units thereof.

14. Polymer composition according to Claim 12 or 13,
**characterized in that**
plasticizer is present in an amount of 5 to 200 parts by mass per 100 parts by mass of polymer.

15. Polymer composition according to any of Claims 12 to 14,
**characterized in that**
at least one of the polymers present is a copolymer of vinyl chloride with one or more monomers selected from the group consisting of vinylidene chloride, vinyl acetate, vinyl propionate, vinyl butyrate, vinyl benzoate, methyl acrylate, ethyl acrylate and butyl acrylate.

16. Moulding or film comprising a polymer composition according to any of Claims 12 to 15.

17. Moulding or film according to Claim 16,
wherein the film or moulding is a floor covering, a wall covering, a hose, a profile, a polymer film, a roofing sheet, a sealing sheet, a cable or wire sheath, a tarpaulin, an advertising banner, synthetic leather, packaging film, a medical article, a toy, a seal, a furnishing article.

## Revendications

1. Ester diheptylique de l'acide furannedicarboxylique, **caractérisé en ce qu'**il présente des propriétés suivantes :
1) sa viscosité propre à 25°C est d'au maximum 120 mPa.s
2) lors de la mesure par un calorimètre différentiel, on n'observe pas de signal de fusion aux températures > 20°C.

2. Ester diheptylique selon la revendication 1, **caractérisé en ce que** sa densité à 20°C est d'au maximum 1,1 g/cm³.

3. Composition de plastifiant qui contient au moins un ester diheptylique selon l'une quelconque des revendications 1 ou 2, celle-ci contenant au moins deux furanne-2,5-dicarboxylates de diheptyle isomères.

4. Composition de plastifiant selon la revendication 3, **caractérisée en ce que** les furanne-2,5-dicarboxylates de diheptyle isomères contiennent des groupes heptyle isomères.

5. Composition de plastifiant selon l'une quelconque des revendications 3 ou 4, **caractérisée en ce qu'**aucun des furanne-2,5-dicarboxylates de diheptyle isomères ne représente une proportion supérieure à 99,9% en poids dans le mélange d'esters.

6. Composition de plastifiant selon la revendication 4, **caractérisée en ce qu'**au moins un des groupes heptyle isomères est choisi parmi : les groupes n-heptyle, 2-méthylhexyle, 3-méthylhexyle, 4-méthylhexyle, 5-méthylhexyle, 2,2-diméthylpentyle, 2,3-diméthylpentyle, 3,3-diméthylpentyle, 2,4-diméthylpentyle, 3-éthylpentyle, 2,2,3-triméthylbutyle et 2-éthyl-3-méthylbutyle.

7. Composition de plastifiant selon au moins l'une quelconque des revendications 3 à 6, **caractérisée en ce qu'**elle contient au moins un plastifiant supplémentaire, choisi dans le groupe formé par les benzoates d'alkyle, les adipates de dialkyle, les esters de glycérol, les esters trialkyliques de l'acide citrique, les esters trialkyliques acylés de l'acide citrique, les trimellitates de trialkyle, les dibenzoates de glycol, les téréphtalates de dialkyle, les phtalates de dialkyle, les dialcanoates d'isosorbide, les esters dialkyliques de l'acide 1,2-cyclohexanedicarboxylique, 1,3-cyclohexanedicarboxylique ou 1,4-cyclohexanedicarboxylique.

8. Procédé pour la préparation d'un ester diheptylique selon la revendication 1 ou 2, comprenant les étapes de procédé consistant à :
a) mettre en contact de l'acide furannedicarboxylique et/ou d'au moins un dérivé de l'acide furannedicarboxylique avec un ou plusieurs alcools aliphatiques comprenant 7 atomes de carbone,
b) chauffer le mélange réactionnel décrit à une température > 50°C et estérification ou, selon le cas, transestérification avec séparation (de préférence thermique) d'au moins une substance de bas poids moléculaire du mélange réactionnel.

9. Procédé pour la préparation d'un ester diheptylique selon la revendication 1 ou 2, comprenant les étapes de procédé consistant à :
a) mettre en contact du 5-hydroxyméthylfurfural et/ou au moins un dérivé du furanne avec un ou plusieurs alcools aliphatiques comprenant 7 atomes de carbone ainsi qu'avec au moins un catalyseur et au moins un composant contenant de l'oxygène,
b) conditionner thermiquement le mélange réactionnel décrit à une température > 0°C et réaliser une estérification par oxydation.

10. Utilisation de la composition de plastifiant selon l'une quelconque des revendications 3 à 7 comme plastifiant pour des polymères.

11. Utilisation d'un ester diheptylique selon la revendication 1 ou 2 dans des adhésifs, des masses de bouchage, des masses de revêtement, des laques, des peintures, des plastisols, des pâtes, des cuirs synthétiques, des revêtements de sol, la protection des bas de caisse, des revêtements de tissus, des tapis ou des encres.

12. Composition de matériau synthétique contenant au moins un ester diheptylique selon la revendication 1 ou 2.

13. Composition de matériau synthétique selon la revendication 12, **caractérisée en ce qu'**elle contient au moins un polymère, choisi parmi le poly(chlorure de vinyle) (PVC), le poly(chlorure de vinylidène) (PVDC), les polyacrylates, en particulier le poly(méthacrylate de méthyle)(PMMA), le poly(méthacrylate d'alkyle) (PAMA), les polymères fluorés, en particulier le poly(fluorure de vinylidène) (PVDF), le polytétrafluoroéthylène (PTFE), le poly(acétate de vinyle) (PVAc), le poly(alcool vinylique) (PVA), les polyvinylacétals, en particulier le polyvinylbutyral (PVB), les polymères de polystyrène, en particulier le polystyrène (PS), le polystyrène expansible (EPS), l'acrylonitrile-styrène-acrylate (ASA), le styrène-acrylonitrile (SAN), l'acrylonitrile-butadiène-styrène (ABS), le copolymère de styrène-anhydride de l'acide maléique (SMA), le copolymère de styrène-acide méthacrylique, les polyoléfines, en particulier le polyéthylène (PE) ou le polypropylène (PP), les polyoléfines thermoplastiques (TPO), le polyéthylène-acétate de vinyle (EVA), les polycarbonates, le poly(téréphtalate d'éthylène) (PET), le poly(téréphtalate de butylène) (PBT), le polyoxyméthylène (POM), le polyamide (PA), le polyéthylèneglycol (PEG), le polyuréthane (PU), le polyuréthane thermoplastique (TPU), les polysulfures (PSu), les biopolymères, en particulier le poly(acide lactique) (PLA), le polyhydroxybutyral (PHB), le poly(acide hydroxyvalérianique) (PHV), le polyester, l'amidon, la cellulose et les dérivés de cellulose, en particulier la nitrocellulose (NC), l'éthylcellulose (EC), l'acétate de cellulose (CA), l'acétate/butyrate de cellulose (CAB), les gommes ou les silicones ainsi que les mélanges ou les copolymères des polymères mentionnés ou de leurs motifs monomères.

14. Composition de matériau synthétique selon la revendication 12 ou 13, **caractérisée en ce que** le plastifiant est contenu en une quantité de 5 à 200 parties en masse par 100 parties en masse de polymère.

15. Composition de matériau synthétique selon l'une quelconque des revendications 12 à 14, **caractérisée en ce qu'**au moins un des polymères contenus est un copolymère de chlorure de vinyle avec un ou plusieurs monomères choisis dans le groupe constitué par le chlorure de vinylidène, l'acétate de vinyle, le propionate de vinyle, le butyrate de vinyle, le benzoate de vinyle, l'acrylate de méthyle, l'acrylate d'éthyle ou l'acrylate de butyle.

16. Corps façonné ou feuille contenant une composition de matériau synthétique selon l'une quelconque des revendications 12 à 15.

17. Corps façonné ou feuille selon la revendication 16, la feuille ou le corps façonné étant un revêtement de sol, un revêtement mural, un flexible, un profilé, un film polymère, une bande de couverture, une bande d'étanchéité, une gaine de câble ou de fil, une bâche, une bannière publicitaire, un cuir synthétique, une feuille d'emballage, un objet médical, un jouet, un joint, un objet d'équipement.
